# EUROPEAN PATENT SPECIFICATION

(11) **EP 4 253 442 B1**
(45) Date of publication and mention of the grant of the patent: **29.04.2026**
(21) Application number: 23190828.6
(22) Date of filing: 27.02.2020
(51) Int. Cl.: C07C 211/14, C07D 207/00, C08G 18/18

(54) **AMINE COMPOSITION USEFUL FOR MAKING STABLE POLYURETHANE FOAM SYSTEMS**
AMINZUSAMMENSETZUNG ZUR HERSTELLUNG VON STABILEN POLYURETHANSCHAUMSYSTEMEN
COMPOSITION D'AMINE UTILE DANS LA FABRICATION DE SYSTÈMES DE MOUSSE DE POLYURÉTHANE STABLE

(30) Priority: 28.02.2019 US 201962811954 P
(43) Date of publication of application: 04.10.2023
(62) Divisional of application: 20710068.6
(73) Proprietor: Evonik Operations GmbH, 45128 Essen (DE)
(72) Inventor: Burdeniuc, Juan Jesus, Colmar, PA 18915 (US); Tobias, James Douglas, Center Valley, PA 18034 (US); Miller, Timothy Joseph, Northampton, PA 18067 (US); Singh, Mayank Pratap, Chesterbrook, PA 19087 (US); VanderSande, David, Allentown, PA 18103 (US)
(74) Representative: Evonik Patent Association

(56) References cited:
- WO-A1-2012/150998
- WO-A1-2014/047230
- WO-A1-2016/020139
- WO-A1-2019/055443
- DD-A1- 279 601
- US-A- 2 716 134
- US-A- 4 474 988
- US-A- 5 438 114
- US-A1- 2009 099 273
- US-A1- 2015 291 506
- US-B2- 6 583 246
- US-B2- 9 000 061
- JULIA L. BILKE ET AL: "On the Two-Ligand Catalytic Asymmetric Deprotonation of N -Boc Pyrrolidine: Probing the Effect of the Stoichiometric Ligand +", JOURNAL OF ORGANIC CHEMISTRY, vol. 73, no. 16, 1 August 2008 (2008-08-01), US, pages 6452 - 6454, XP055697358, ISSN: 0022-3263, DOI: 10.1021/jo8010655
- MOORE G G I ET AL: "Electrochemical fluorination of aminoalkyl ethers", JOURNAL OF FLUORINE CHEMISTRY, ELSEVIER, NL, vol. 32, no. 1, 1 June 1986 (1986-06-01), pages 41 - 76, XP026619622, ISSN: 0022-1139, [retrieved on 19860601], DOI: 10.1016/S0022-1139(00)80506-0
- EDWARD G. KNAPICK ET AL: "Preparation of N,N'-n-alkyl-dimethyl-1-omega-alkanediames", SYNTHESIS, vol. 1, 1 January 1985 (1985-01-01), pages 58 - 60, XP055697960
- DATABASE REGISTRY [online] CHEMICAL ABSTRACTS SERVICE, COLUMBUS, OHIO, US; 8 March 2016 (2016-03-08), "2,2'-oxybis[N-ethyl-N-methyl-ethanamine", XP002810469, Database accession no. 1881998-28-2

## Description

### FIELD OF THE INVENTION

The field of invention is the composition and application of catalysts useful for the production of insulating polyurethane foam produced with blowing agents containing a halogen.

### BACKGROUND OF THE INVENTION

Polyurethane foam compositions are typically prepared by reacting an isocyanate and a premix which consists of isocyanate-reactive components such as a polyol. The premix optionally also contains other components such as water, flame retardants, blowing agents, foam-stabilizing surfactants, and catalysts to promote the reactions of isocyanate with polyol to make urethane, with water to make CO₂ and urea, and with excess isocyanate to make isocyanurate (trimer).

The blowing agent in the premix is usually a liquid or gas with a boiling point sufficiently low to be vaporized by the heat released during the polymerization reaction. Examples of blowing agents useful in the production of insulating polyurethane foam include but are not limited to hydrofluorocarbons (HFCs), hydrofluoroolefins (HFOs), hydrofluorochloroolefins (HFCOs), hydrochlorofluorocarbons (HCFCs), formates, ketones such as acetone and hydrocarbons.

Unlike simple hydrocarbons, such as pentane, halogen containing molecules such as chrolofluorocarbons (CFCs), hydrochlorofluorocarbons (HCFCs) and hydrofluorocarbons (HFCs) are far less flammable and safer to use in foam production. However, they either harm the ozone layer or contribute to global warming in other ways. In contrast, HFOs are very efficient and environmentally friendly blowing agents with a much lower global warming potential (GWP). However, decomposition of HFO can happen in a polyol premix formulation having an amine catalyst. Considering the wide use of amine catalysts in polyurethane foam production, this has limited the use of HFOs.

The proper selection and combination of the components in the polyol premix and the isocyanate can be useful for the production of polyurethane foam that is spray applied, poured in place, and used in applications such as refrigerators, freezers, hot water heaters, insulation panels, garage doors, entry doors, and other various applications where insulation is desired.

For some of these applications, the premix is stored for one day up to one year before being reacted with isocyanate to generate polyurethane foam. This is common in sprayfoam applications, where drums of polyol premix and isocyanate are shipped to field locations for on-site application.

Thus, it is desirable for the premix of an insulating foam formulation to be both chemically and physically stable. However, the catalysts that are useful to promote the polyurethane reaction can also result in undesired reactions with the blowing agents present in the premix resulting in reduced storage stability. These undesired reactions are prevalent in blowing agents that contain halogens, and are especially problematic in blowing agents containing halogens bonded to olefinic carbons. Common amine catalysts useful for the production of polyurethane foam include tertiary amines, such as N,N,N',N",N"-pentamethyldiethylenetriamine (available from Evonik as Polycat^{®}-5) or 1,4-diazabicyclo[2.2.2]octane (available in solution from Evonik as Dabco^{®}33LV) which are known to accelerate the urethane reaction promoting the formation of polyurethane polymers. However, tertiary amines are also known to react with halogen containing organic compounds causing deactivation of the tertiary amine catalysts resulting in a net decrease in the kinetics of the polymerization process. Reaction between tertiary amine and halogen containing organic compounds occurs more rapidly when the halogen atom is bound to an olefinic carbon because halogen-substituted olefins are susceptible to nucleophillic attack by tertiary amines. This results in a fast deactivation of the tertiary amine catalysts rendering the premix not active enough for reaction with the isocyanate. Deactivation of tertiary amine by reaction with halogen containing compounds can also occur in halogen containing aliphatic compounds via formation of a quaternary ammonium salt or dehydrohalogenation, both pathways resulting in tertiary amine deactivation. One way to minimize the deactivation of the amine catalysts is by selecting tertiary amines with bulky substituents to minimize the nucleophillic character of the amine center reducing the kinetics of these decomposition processes. However, the limitation of this approach is that sterically hindered amines are also poor catalysts for the urethane reaction requiring high use levels of tertiary amines and in many instances large use levels of amines are not sufficient to achieve the desired reaction speed. Another common way to mitigate the deactivation of tertiary amine by these processes is by combining the tertiary amine catalyst with an organic carboxylic acid to produce a tertiary ammonium carboxylate salt. The disadvantage of this approach is that the salt is substantially less active than the free tertiary amine resulting in slow reaction rates that can impact both productivity and product quality. Thus, the present invention is directed to processes, compositions, and products having new catalyst compositions able to produce a premix that is sufficiently stable for storage without the limitation of excessive catalyst deactivation. It has been found that certain catalyst compositions can be successfully employed when the chemical architecture of the catalysts is selected so that interaction between the hydrofluororolefin blowing agent is minimized but without sacrificing its interation with water or an alcohol functionality needed to promote the urethane polymerization.

US9000061 discloses a method to make pour in place polyurethane foam as well as polyol premixes comprising 1-chloro-3,3,3-trifluoropropene (HFCO-1233zd) with one or more co-blowing agents. Some of the useful catalysts listed in the specification structurally related to the catalyst of the invention included N, N, N', N", N"-pentamethyltriethylenediamine and N, N, N', N", N"-pentaethyltriethylenediamine. The compound N, N, N', N", N"-pentamethyltriethylenediamine is commercially known as Polycat^{®}-5 and as shown in Example 11, excessive deterioration of the hydrofluoroolefin leads to deactivation of the catalyst and causes sagging of the polyurethane mass in spraying systems that have been aged for two weeks or more. The compound N, N, N', N", N"-pentamethyltriethylenediamine is considered a sterically hindered blowing catalyst. To solve the problem of catalyst deactivation observed when using Polycat^{®}-5, full substitution of Me-groups for Ethyl-groups is suggested. However, this approach brings excessive steric hindrance and deactivation of the catalysts rendering it essentially ineffective even at high use levels. This is in part by the introduction of a relatively large alkyl group at the central nitrogen atom of the diethylene triamine backbone. Thus, on a related matter N, N"-diisopropyl-N, N', N"-trimethyldiethylenetriamine is presented as an effective catalyst of this invention while the ethyl substituted analogue N, N"-diisopropyl-N, N', N"-triethyldiethylenetriamine was completely ineffective as shown in Example 14.

US9453115 discloses polyurethane and polyisocyanurate foams as well as their preparation methods which included the use of polyol-based foaming mixtures containing hydrohaloolefin blowing agents in the presence of a catalyst which is an adduct of an amine and an organic acid. The inventive polyol premix composition contains a catalyst which is an adduct between an amine and an organic acid. Multiple examples of tertiary amines are considered but particularly important are the sterically hindered amine adducts with organic acids including carboxylic acids, dicarboxylic acids, phosphinic acids, phosphonic acids, sulfonic acids, etc. The stabilization of the polyol premix containing the hydrohaloolefin is given by virtue of reducing the overall alkalinity of the premix with an acid. The limitation of this approach is that the use of acids in the presence of tertiary amine catalysts substantially reduces their ability to promote the polymerization process. Also, tertiary amine salts are typically insoluble or have tendency to precipitate from liquid mixtures making the process of foam making more difficult. In addition, many of the acids utilized are corrosive and can induce damage of mechanical equipment.

US9051442 discloses a method to make polyurethane foam and in particular closed cell rigid polyurethane foam made with a polyol premix which comprises a combination of a hydrohaloolefin physical blowing agent, a polyol, a silicone surfactant and a non-amine catalyst used alone or in combination with an amine catalyst. The foam is characterized by a fine and uniform cell structure and little or no foam collapse. The disclosure teaches that amines that are relatively stable with hydrohaloolefins are generally not sufficiently active to provide the necessary foam reactivity. The disclosure also teaches that there is a large number of amine catalysts that can be identified as sufficiently active to produce acceptable foam reactivity but that these catalysts are not suitable for use with hydrohaloolefins due to the formation of fluoride ion. Because of this limitation a catalyst system comprising at least a first metal and at least a second metal and at least one amine catalyst with a pKa of no less than ten is suggested as a plausible solution to this problem, where the at least first metal and the at least second metal are typically bismuth nitrate, lead 2-ethylhexanoate, lead benzoate, lead naphthanate, ferric chloride, antimony trichloride, antimony glycolate, tin salts of carboxylic acids, dialkyl tin salts of carboxylic acids, potassium acetate, potassium octoate, potassium 2-ethylhexanoate, potassium salts of carboxylic acids, zinc salts of carboxylic acids, zinc 2-ethylhexanoate, glycine salts, dibutyltin dilaureate, sodium N-(2-hydroxy-5-nonylphenol)methyl-N-methylglycinate, tin(II) 2-ethylhexanoate and combinations thereof. The limitation in this approach is the introduction of heavy metals many of which are toxic or hydrolytically unstable or in the case of the alkali metal salts of carboxylic acid induction of other reactions such as trimerization to form polyisocyanurates makes the processing aspect more difficult because there is not an easy way to balance the gelling and blowing reaction processes thereby compromising the final properties of the polyurethane product.

US9133306 discloses a polymeric amine composition having a multiplicity of tertiary amine groups and a method to make the composition. The composition is used as an amine-epoxy curing agent or alternatively as a chain extender or catalyst in polyurethane applications. The disclosure does not teach and does not show how any particular combination within the genus of the disclosure could be used in hydrohaloolefins-polyol premixes and systems. Furthermore, the preferred compositions having primary and secondary amine groups are expected to react very rapidly with hydrohaloolefins.

US2015/0197614A1 discloses a polyol premix composition which includes a blowing agent having a halogenated hydrohalolefin, a polyol, a catalyst composition and an antioxidant. The role of the antioxidant is to stabilize the hydrohaloolefin when present in the system together with amine catalyst. The presence of the antioxidant helps enable to some extent the use of tertiary amine catalysts that otherwise would lead to system deactivation. This approach nevertheless requires a substantial amount of antioxidant which does not play any other functional role in the polyurethane process. This approach also requires substantial amounts of antioxidants to have a positive impact on foam kinetics after ageing and it does not address the question regarding the structural requirements that a catalyst needs to meet to function in these haloolefin containing premixes.

US2016/0130416A1 discloses a stable polyol premix composition comprising hydrohaloolefin blowing agent, a polyol, a surfactant, and a catalyst composition comprising a substituted imidazole having a C₂ or greater substitution at the N1 nitrogen atom.

JP2014105288 discloses a composition for polyurethane foam manufacturing including tertiary amine catalysts comprising an amine compound represented by the general formula R₁R₂N-(CH₂)ₘ-[X-(CH₂) ₙ-]-Z where R₁ and R₂ are each independently an alkyl or hydroxyalkyl group having 2 to 8 carbon atoms and where Z represents -OH or - NR₃R₄ and X is -O- or -NR₅- where R₃ and R₄ represents an alkyl or hydroxyalkyl group having 2-8 carbon atoms where R₅ represents an alkyl or hydroxyalkyl having 2 to 8 carbon atoms. Because the smallest substituent in this composition is essentially an ethyl group, the steric hindrance around the nitrogen atoms is too excessive to provide sufficient catalytic activity to make these compounds useful in foam applications. Thus, these compounds can provide stable hydrohaloolefin-polyol stable systems but the catalytic activity will be insufficient even at very high use levels as illustrated in example 14 in the experimental section. WO2012/150998 discloses a polyol premix composition comprising a blowing agent, a polyol, a surfactant and a catalyst composition comprising an oxygen-containing amine catalyst.

Thus, there is a need in the art for making polyurethane foam using low GWP blowing agents such as hydrohaloolefins with catalysts that are able to provide sufficient kinetics after ageing of the polyol premix systems. The catalysts presented in this invention can offer the right balance for making foam even in applications where high foam speed rise is needed (spray foam, for example) without sacrificing the shelf life of the hydrohaloolefin-polyol premix system.

### BRIEF SUMMARY OF THE INVENTION

The instant invention can solve problems associated with conventional foam precursors by allowing the use of the inventive catalysts thereby improving the storage stability of an isocyanate reactive mixture comprising various types of polyols known in the art, and various blowing agents including pentane, halogen containing molecules such as chrolofluorocarbons (CFCs), hydrochlorofluorocarbons (HCFCs) and hydrofluorocarbons (HFCs), hydrofluoroolefins (HFOs), hydrochloroolefins (HCOs), hydrochlorofluoroolefins (HCFOs), haloolefins and hydrohaloolefins. The invention is particularly useful when using HFOs and haloolefin blowing agents.

The present invention provides a polyurethane catalyst and a polyol premix composition having the following benefits: a) minimize blowing agent, flame retardant and polyester polyol degradation in the premix mixture; b) minimize HFOs, haloolefins and hydrohaloolefins degradation of the premix allowing the use of low GWP blowing agents; c) minimize or eliminate emissions associated with the catalyst; d) provide optimum catalytic activity and foam physical properties.

It is disclosed that a catalyst composition is preferably defined as at least one compound with a general formula I: 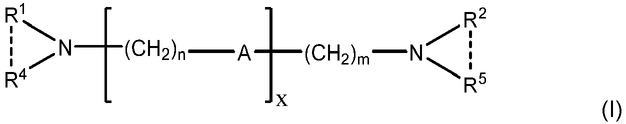 where A is N-R³, where R₃ is C₁-C₈ linear or branched and preferably C₁-C₃ alkyl group, x = 0-6, n and m are each independently 1 to 6, R¹ and R² are each independently C₂-C₈ alkyl and preferably C₂-C₃ alkyl group, and R⁴ and R⁵ are -CH₃ groups.

It is disclosed that a catalyst composition is preferably defined as at least one compound with a general formula I: 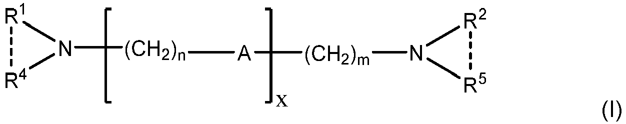 where A = O, x = 0-6, n and m are each independently 1 to 6, R¹ and R² are each independently C₂-C₈ alkyl and preferably C₂-C₃ alkyl group, and R⁴ and R⁵ are -CH₃ groups.

It is disclosed that a catalyst composition is preferably defined as at least one compound with a general formula I: 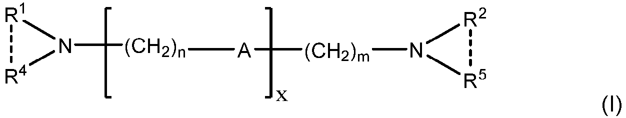 where A = O or N-R³, R³ is C₁-C₈ linear or branched, and where N(R¹---R⁴) and N(R²-R⁵) each independently represent a C₃-C₇ ring amine moiety of the type:

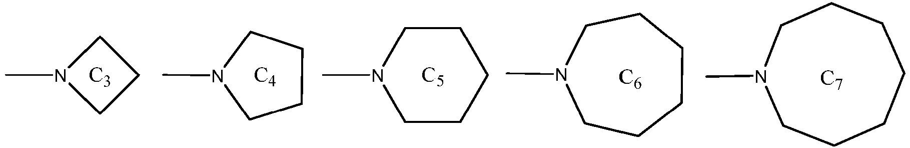

The invention specifically relates to a catalyst composition comprising a compound selected from a list of compounds that fall within general formula I, i.e. a compound selected from the group consisting of N, N'-dipropyl-N, N'-dimethyl-bis(aminoethyl)ether; N, N'-diisopropyl-N, N'-dimethyl-bis(aminoethyl)ether; N, N'-dibutyl-N, N'-dimethyl-bis(aminoethyl)ether; N, N'-diisobutyl-N, N'-dimethyl-bis(aminoethyl)ether; N, N'-disecbutyl-N, N'-dimethyl-bis(aminoethyl)ether; N, N'-ditertbutyl-N, N'-dimethyl-bis(aminoethyl)ether; N, N'-dipentyl-N, N'-dimethyl-bis(aminoethyl)ether; N, N'-ditertpentyl-N, N'-dimethyl-bis(aminoethyl)ether; N, N'-dineopentyl-N, N'-dimethyl-bis(aminoethyl)ether; N, N'-disecpentyl-N, N'-dimethyl-bis(aminoethyl)ether; N, N'-di(3-pentyl)-N, N'-dimethyl-bis(aminoethyl)ether; N, N'-disecisoopentyl -N, N'-dimethyl-bis(aminoethyl)ether; N, N'-dihexyl-N, N'-dimethyl-bis(aminoethyl)ether; N, N'-diisohexyl-N, N'-dimethyl-bis(aminoethyl)ether; and N, N'-dineohexyl-N, N'-dimethyl-bis(aminoethyl)ether.

In an exemplary embodiment, a process includes providing a premix comprising at least one catalyst component of inventive composition and contacting the pre-mix containing the new compositions with at least one of the blowing agents including HFOs such as hydrofluoroolefins, and hydrofluorochloroolefins.

In another exemplary embodiment, a polyurethane composition includes a polyol component, a catalyst composition, and an isocyanate component. The catalyst composition includes at least one component of the inventive composition.

In another exemplary embodiment, a polyurethane product is formed from the polyurethane composition that includes at least one catalyst component of inventive composition, a polyol component and an isocyanate component.

In another exemplary embodiment, a catalyst includes at least one catalyst component of the general formula I of the new composition and a metal catalyst or a tertiary amine catalyst having or not an isocyanate reactive group, or a combination thereof.

Other features and advantages of the present invention will be apparent from the following more detailed description of the preferred embodiment, taken in conjunction with the accompanying drawings which illustrate, by way of example, the principles of the invention.

### BRIEF DESCRIPTION OF THE DRAWINGS

Figure 1 is a graphical representation in terms of seconds v. mm of the rate of rise for foam made in accordance with Example 10 at 0 week and 4 weeks thermal ageing.
Figure 2 is a graphical representation in terms of seconds v. mm of the rate of rise for foam made in accordance with Example 11 at 0 week and 4 weeks thermal ageing.
Figure 3 is a graphical representation in terms of seconds v. mm of the rate of rise for foam made in accordance with Example 12 at 0 week and 4 weeks thermal ageing.
Figure 4 is a graphical representation of the time in seconds to 80% maximum height for foams made in accordance with Examples 9, 11 and 12 at 0 week, 1 week, 2 weeks and 4 weeks thermal ageing.
Figure 5 is a graphical representation in terms of the foam height (mm) *vs.* time (seconds) for a foam made with Polycat^{®}5 (pentamethyltriethylenediamine) in accordance with Example 13 at the initial stage, 2 weeks and 4 weeks thermal ageing.
Figure 6 is a graphical representatios in terms of the foam height (mm) *vs*. time (seconds) for a foam made with the catalyst of Example 2, at the initial stage, 2 weeks and 4 weeks thermal ageing.
Figure 7 is a graphical representation in terms of the foam height (mm) *vs*. time (seconds) for a foam made with the catalyst of Example 3, at the initial stage, 2 weeks and 4 weeks thermal ageing.
Figure 8 is a graphical representation illustrating the impact on HFO-based system stability for catalysts of example 5.
Figure 9 is a graphical representation illustrating the impact on HFO-based system stability for catalysts of example 4.
Figure 10 is a graphical representation illustrating the impact on HFO-based system stability for catalysts of example 6.
Figure 11 is a graphical representation illustrating the impact on HFO-based system stability for catalysts of example 7.
Figure 12 is a bar diagram representing the stability after aging at 50 °C for the catalysts listed in Table 8.
Figure 13 is a graphical representation in terms of seconds v. mm of the rate of rise for foam made in accordance with Example 16 at 0 days and 5 days.
Figure 14 is a graphical representation in terms of seconds v. mm of the rate of rise for foam made in accordance with Example 17 at 0 week and 4 weeks.
Figure 15 is a graphical representation in terms of seconds v. mm of the rate of rise for foam made in accordance with Example 18 at 0 week and 4 weeks.
Figure 16 is a graphical representation of the time in seconds to 80% maximum height for foams made with various catalysts at 0 week, 1 week, 2 weeks and 4 weeks.
Figure 17 is a graphical representation in terms of seconds v. mm of the rate of rise for foam made in accordance with Example 19 at 0 week and 4 weeks.
Figure 18 is a graphical representation in terms of seconds v. mm of the rate of rise for foam made in accordance with Example 20 at 0 week and 4 weeks.

### DEFINITIONS

The following definitions are provided in order to aid those skilled in the art in understanding the detailed description of the present invention.
PUR - Polyurethane.
Isocyanate Index - The actual amount of polyisocyanate used divided by the theoretically required stoichiometric amount of polyisocyanate required to react with all the active hydrogen in the reaction mixture, multiplied by 100. Also known as (Eq NCO/Eq of active hydrogen)x100.
pphp - parts by weight per hundred weight parts polyol.
Polycat^{®}-5 - A commercial catalyst supplied by Evonik Corporation with a chemical name pentamethyldiethylenetriamine
HFO - hydrofluoroolefin

### DETAILED DESCRIPTION OF THE INVENTION

The present invention is directed to a new haloolefin containing polyol premix having a catalyst composition. The disclosure relates to a catalyst composition is defined as at least one compound with a general formula I: 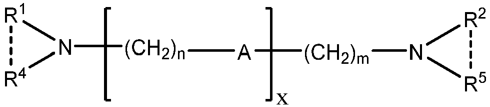 where A is N-R³, where R³ is C₁-C₈ linear or branched and preferably C₁-C₃ alkyl group, x = 0-6, n and m are each independently 1 to 6, R¹ and R² are each independently C₂-C₈ alkyl and preferably C₂-C₃ alkyl group, and R⁴ and R⁵ are -CH₃ groups.

The disclosure further relates to catalyst composition is preferably defined as at least one compound with a general formula I: 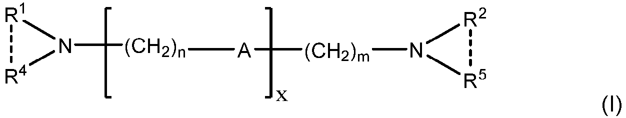 where A = O, x = 0-6, n and m are each independently 1 to 6, R¹ and R² are each independently C₂-C₈ alkyl and preferably C₂-C₃ alkyl group, and R⁴ and R⁵ are -CH₃ groups.

It is further disclosed that the catalyst composition is preferably defined as at least one compound with a general formula I: 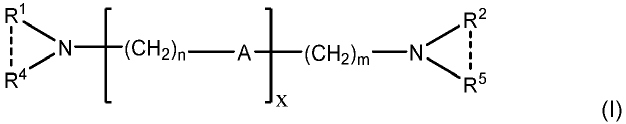 where A = O or N-R³ as defined above and where N(R¹---R⁴) and N(R²---R⁵) each independently represent a C₃-C₇ ring amine moiety of the type:

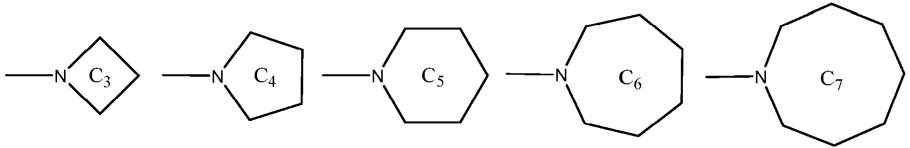

In another embodiment, the catalyst includes at least one catalyst component of the inventive composition and a metal catalyst or a tertiary amine catalyst having or not an isocyanate reactive group, or a combination thereof.

The present invention provides a polyurethane catalyst composition having the following benefits: a) minimize blowing agent, flame retardant and polyester polyol degradation in the premix mixture; b) minimize HFOs, haloolefins and hydrohaloolefins degradation of the premix allowing the use of low GWP blowing agents; c) minimize or eliminate emissions associated with the catalyst; and d) provide optimum catalytic activity and foam physical properties.

Also, the present invention in one embodiment provides a method for preparing a polyurethane foam which comprises contacting at least one polyisocyanate with at least one active hydrogen-containing compound in the presence of at least one blowing agent and an effective amount of the inventive catalyst composition.

In another embodiment, the method for preparing a polyurethane foam comprises contacting at least one polyisocyanate with at least one active hydrogen-containing compound in the presence of at least one blowing agent and an effective amount of the inventive catalyst composition in combination with a metal catalyst, a tertiary amine having or not an isocyanate reactive group, or a combination of a metal catalyst and a tertiary amine having or not an isocyanate reactive group.

Additionally, polyurethane foams can be produced with the catalyst system and compositions of the present invention by several methods known within the art.

Several types of ranges are disclosed in the present invention. These include, but are not limited to, a range of temperatures; a range of number of atoms; a range of foam density; a range of Isocyanate Index; and a range of pphp for the blowing agent, water, surfactant, flame retardant, and an inventive catalyst composition as defined above. Each possible number that such a range could reasonably encompass, as well as any sub-ranges and combinations of sub-ranges encompassed therein are the subject of the present invention. For example, for a chemical moiety having a certain number of carbon atoms, every possible number that such a range could encompass, consistent with the disclosure herein are the subject of the present invention.

For example, the disclosure that R¹ and R² are each independently C₂-₈ respectively alkyl linear or branched, alkenyl linear or branched mean for example that an alkyl group having up to 8 carbon atoms, or in alternative language a C₂-₈ alkyl group, as used herein, refers to a "R²" or "R³" group that can be selected independently from an alkyl group having 2, 3, 4, 5, 6, 7 or 8 carbon atoms, as well as any range between these two numbers (for example, a C₂ to C₄ alkyl group), and also including any combination of ranges between these two numbers (for example, a C₂ to C₃ and C₄ to C₆ alkyl group).

Similarly, another representative example follows for the parts by weight of the inventive catalyst composition per hundred weight parts of the at least one active hydrogen-containing compound in a composition or a foam formulation. If the at least one active hydrogen-containing compound is an at least one polyol, the parts by weight per hundred weight parts polyol is abbreviated as pphp. Hence, by the disclosure that the inventive catalyst composition is present in an amount from about 0.05 to about 10 pphp, for example, the pphp can be selected from about 0.05, about 0.06, about 0.07, about 0.08, about 0.09, about 0.1, about 0.2, about 0.3, about 0.4, about 0.5, about 0.6, about 0.7, about 0.8, about 0.9, about 1, about 2, about 3, about 4, about 5, about 6, about 7, about 8, about 9, or about 10. Likewise, all other ranges disclosed herein should be interpreted in a manner similar to these two examples.

Any individual members of any such group, including any sub-ranges or combinations of sub-ranges within the group may be excluded. Further, any individual substituents, analogs, compounds, ligands, structures, or groups thereof, or any members of a claimed group may be excluded.

In another aspect of the invention, the catalyst compositions can be used to make rigid foams (foam that is unable to bend or be forced out of shape) having a density of about 8.0 kg/m³ to about 80 Kg/m³ (about 0.5 lb/ft³ to about 5 lb/ft³₎, about 16 Kg/m³ to about 64 Kg/m³ (about 1 lb/ft³ to about 4 lb/ft³) and in some cases about 32 Kg/m³ to about 48 Kg/m³ (about 2 lb/ft³ to about 3 lb/ft³). The catalyst compositions can be used to make close celled spray foam having desirable physical properties including dimensional stability, adhesion, friability, thermal insulation and compression strengths. In a further aspect, the catalyst compositions can be used to make spray foams having a density of 8.0 Kg/m³ to about 80 Kg/m³ (about 0.5 lb/ft³ to about 5 lb/ft³), about 16 Kg/m³ to about 64 Kg/m³ (about 1 lb/ft³ to about 4 lb/ft³) and in some cases about 32 Kg/m³ to about 48 Kg/m³ (about 2 lb/ft³ to about 3 lb/ft³). Density can be measured in accordance with ASTM D3574 Test A.

It is disclosed that the catalyst composition as defined in formula I preferably comprises at least one member selected from the group consisting of N, N"-diethyl-N, N', N"-trimethyl(diethylenetriamine); N, N"-dipropyl-N, N', N"-trimethyl(diethylenetriamine); N, N"-diisopropyl-N, N', N"-trimethyl(diethylenetriamine); N, N"-dibutyl-N, N', N"-trimethyl(diethylenetriamine); N, N"-diisobutyl-N, N', N"-trimethyl(diethylenetriamine); N, N"-disecbutyl-N, N', N"-trimethyl(diethylenetriamine); N, N"-ditertbutyl-N, N', N"-trimethyl(diethylenetriamine); N, N"-dipentyl-N, N', N"-trimethyl(diethylenetriamine); N, N"-diisopentyl-N, N', N"-trimethyl(diethylenetriamine); N, N"-ditertpentyl-N, N', N"-trimethyl(diethylenetriamine); N, N"-dineopentyl-N, N', N"-trimethyl(diethylenetriamine); N, N"-disecpentyl-N, N', N"-trimethyl(diethylenetriamine); N, N"-di(3-pentyl)-N, N', N"-trimethyl(diethylenetriamine); N, N"-disecisoopentyl-N, N', N"-trimethyl(diethylenetriamine); N, N"-dihexyl-N, N', N"-trimethyl(diethylenetriamine); N, N"-diisohexyl-N, N', N"-trimethyl(diethylenetriamine); N, N"-dineohexyl-N, N', N"-trimethyl(diethylenetriamine); N, N"'-diethyl-N, N', N",N‴-tetramethyl(triethylenetetraamine); N, N"'-dipropyl-N, N', N",N‴-tetramethyl(triethylenetetraamine); N, N"'-diisopropyl-N, N', N",N‴-tetramethyl(triethylenetetraamine); N, N"'-dibutyl-N, N', N",N‴-tetramethyl(triethylenetetraamine); N, N"'-disobutyl-N, N', N",N‴-tetramethyl(triethylenetetraamine); N, N‴-disecbutyl-N, N', N",N‴-tetramethyl(triethylenetetraamine); N, N"'-ditertbutyl-N, N', N",N‴-tetramethyl(triethylenetetraamine); N, N"'-dipentyl-N, N', N",N‴-tetramethyl(triethylenetetraamine); N, N"'-diisopentyl-N, N', N",N‴-tetramethyl(triethylenetetraamine); N, N"'-ditertpentyl-N, N', N",N‴-tetramethyl(triethylenetetraamine); N, N"'-dineopentyl-N, N', N",N‴-tetramethyl(triethylenetetraamine); N, N"'-disecpentyl-N, N', N",N‴-tetramethyl(triethylenetetraamine); N, N"'-di(3-pentyl)-N, N', N",N‴-tetramethyl(triethylenetetraamine); N, N"'-disecisopentyl-N, N', N",N‴-tetramethyl(triethylenetetraamine); N, N"'-dihexyl-N, N', N",N‴-tetramethyl(triethylenetetraamine); N, N"'-diisohexyl-N, N', N",N‴-tetramethyl(triethylenetetraamine); N, N"'-dineohexyl-N, N', N",N‴-tetramethyl(triethylenetetraamine); N, N"-diethyl-N, N', N"-trimethyl(dipropylenetriamine); N, N"-dipropyl-N, N', N"-trimethyl(dipropylenetriamine); N, N"-diisopropyl-N, N', N"-trimethyl(dipropylenetriamine); N, N"-dibutyl-N, N', N"-trimethyl(dipropylenetriamine); N, N"-diisobutyl-N, N', N"-trimethyl(dipropylenetriamine); N, N"-disecbutyl-N, N', N"-trimethyl(dipropylenetriamine); N, N"-ditertbutyl-N, N', N"-trimethyl(dipropylenetriamine); N, N"-dipentyl-N, N', N"-trimethyl(dipropylenetriamine); N, N"-diisopentyl-N, N', N"-trimethyl(dipropylenetriamine); N, N"-ditertpentyl-N, N', N"-trimethyl(dipropylenetriamine); N, N"-dineopentyl-N, N', N"-trimethyl(dipropylenetriamine); N, N"-disecpentyl-N, N', N"-trimethyl(dipropylenetriamine); N, N"-di(3-pentyl)-N, N', N"-trimethyl(dipropylenetriamine); N, N"-disecisoopentyl-N, N', N"-trimethyl(dipropylenetriamine); N, N"-dihexyl-N, N', N"-trimethyl(dipropylenetriamine); N, N"-diisohexyl-N, N', N"-trimethyl(dipropylenetriamine); N, N"-dineohexyl-N, N', N"-trimethyl(dipropylenetriamine); N, N"'-diethyl-N, N', N",N‴-tetramethyl(tripropylenetetraamine); N, N"'-dipropyl-N, N', N",N‴-tetramethyl(tripropylenetetraamine); N, N"'-diisopropyl-N, N', N",N‴-tetramethyl(tripropylenetetraamine); N, N"'-dibutyl-N, N', N",N‴-tetramethyl(tripropylenetetraamine); N, N"'-disobutyl-N, N', N",N‴-tetramethyl(tripropylenetetraamine); N, N"'-disecbutyl-N, N', N",N‴-tetramethyl(tripropylenetetraamine); N, N‴-ditertbutyl-N, N', N",N‴-tetramethyl(tripropylenetetraamine); N, N"'-dipentyl-N, N', N",N‴-tetramethyl(tripropylenetetraamine); N, N‴-diisopentyl-N, N', N",N‴-tetramethyl(tripropylenetetraamine); N, N"'-ditertpentyl-N, N', N",N‴-tetramethyl(tripropylenetetraamine); N, N"'-dineopentyl-N, N', N",N‴-tetramethyl(tripropylenetetraamine); N, N"'-disecpentyl-N, N', N",N‴-tetramethyl(tripropylenetetraamine); N, N"'-di(3-pentyl)-N, N', N",N‴-tetramethyl(tripropylenetetraamine); N, N"'-disecisopentyl-N, N', N",N‴-tetramethyl(tripropylenetetraamine); N, N"'-dihexyl-N, N', N",N‴-tetramethyl(tripropylenetetraamine); N, N"'-diisohexyl-N, N', N",N‴-tetramethyl(tripropylenetetraamine); and N, N"'-dineohexyl-N, N', N",N‴-tetramethyl(tripropylenetetraamine). Such compounds can be employed individually or in any combination thereof.

The inventive catalyst composition as comprises at least one member selected from the group consisting of N, N'-diethyl-N, N'-dimethyl-bis(aminoethyl)ether; N, N'-dipropyl-N, N'-dimethyl-bis(aminoethyl)ether; N, N'-diisopropyl-N, N'-dimethyl-bis(aminoethyl)ether; N, N'-dibutyl-N, N'-dimethyl-bis(aminoethyl)ether; N, N'-diisobutyl-N, N'-dimethyl-bis(aminoethyl)ether; N, N'-disecbutyl-N, N'-dimethyl-bis(aminoethyl)ether; N, N'-ditertbutyl-N, N'-dimethyl-bis(aminoethyl)ether; N, N'-dipentyl-N, N'-dimethyl-bis(aminoethyl)ether; N, N'-dibutyl-N, N'-diisopentyl-bis(aminoethyl)ether; N, N'-ditertpentyl-N, N'-dimethyl-bis(aminoethyl)ether; N, N'-dineopentyl-N, N'-dimethyl-bis(aminoethyl)ether; N, N'-disecpentyl-N, N'-dimethyl-bis(aminoethyl)ether; N, N'-di(3-pentyl)-N, N'-dimethyl-bis(aminoethyl)ether; N, N'-disecisoopentyl -N, N'-dimethyl-bis(aminoethyl)ether; N, N'-dihexyl-N, N'-dimethyl-bis(aminoethyl)ether; N, N'-diisohexyl-N, N'-dimethyl-bis(aminoethyl)ether; and N, N'-dineohexyl-N, N'-dimethyl-bis(aminoethyl)ether. Such compounds can be employed individually or in any combination thereof.

In another embodiment of the invention, the inventive catalyst composition preferably comprises at least one member selected from the group consisting of bis(2-azetidinoethyl)ether; bis(2-pyrrolidinoethyl)ether; bis(2-piperidinodinoethyl)ether; bis(2-azepanoethyl)ether; bis(2-azocanoethyl)ether,and the like. Such compounds can be employed individually or in any combination thereof.

It is further disclosed that the catalyst composition as defined in formula I preferably comprises at least one member selected from the group consisting of N, N"-diethyl-N, N', N"-trimethyl(diethylenetriamine); N, N"-dipropyl-N, N', N"-trimethyl(diethylenetriamine); N, N"-diisopropyl-N, N', N"-trimethyl(diethylenetriamine); N, N"-dibutyl-N, N', N"-trimethyl(diethylenetriamine); N, N"-diisobutyl-N, N', N"-trimethyl(diethylenetriamine); N, N"-disecbutyl-N, N', N"-trimethyl(diethylenetriamine); N, N"-ditertbutyl-N, N', N"-trimethyl(diethylenetriamine); N, N"-dipentyl-N, N', N"-trimethyl(diethylenetriamine); N, N"-diisopentyl-N, N', N"-trimethyl(diethylenetriamine); N, N"-ditertpentyl-N, N', N"-trimethyl(diethylenetriamine); N, N"-dineopentyl-N, N', N"-trimethyl(diethylenetriamine); N, N"-disecpentyl-N, N', N"-trimethyl(diethylenetriamine); N, N"-di(3-pentyl)-N, N', N"-trimethyl(diethylenetriamine); N, N"-disecisoopentyl-N, N', N"-trimethyl(diethylenetriamine); N, N"-dihexyl-N, N', N"-trimethyl(diethylenetriamine); N, N"-diisohexyl-N, N', N"-trimethyl(diethylenetriamine); N, N"-dineohexyl-N, N', N"-trimethyl(diethylenetriamine); N, N"'-diethyl-N, N', N",N‴-tetramethyl(triethylenetetraamine); N, N"'-dipropyl-N, N', N",N‴-tetramethyl(triethylenetetraamine); N, N"'-diisopropyl-N, N', N",N‴-tetramethyl(triethylenetetraamine); N, N"'-dibutyl-N, N', N",N‴-tetramethyl(triethylenetetraamine); N, N"'-disobutyl-N, N', N",N‴-tetramethyl(triethylenetetraamine); N, N"'-disecbutyl-N, N', N",N‴-tetramethyl(triethylenetetraamine); N, N"'-ditertbutyl-N, N', N",N‴-tetramethyl(triethylenetetraamine); N, N"'-dipentyl-N, N', N",N‴-tetramethyl(triethylenetetraamine); N, N"'-diisopentyl-N, N', N",N‴-tetramethyl(triethylenetetraamine); N, N"'-ditertpentyl-N, N', N",N‴-tetramethyl(triethylenetetraamine); N, N"'-dineopentyl-N, N', N",N‴-tetramethyl(triethylenetetraamine); N, N"'-disecpentyl-N, N', N",N‴-tetramethyl(triethylenetetraamine); N, N"'-di(3-pentyl)-N, N', N",N‴-tetramethyl(triethylenetetraamine); N, N"'-disecisopentyl-N, N', N",N‴-tetramethyl(triethylenetetraamine); N, N‴-dihexyl-N, N', N",N‴-tetramethyl(triethylenetetraamine); N, N"'-diisohexyl-N, N', N",N‴-tetramethyl(triethylenetetraamine); N, N"'-dineohexyl-N, N', N",N‴-tetramethyl(triethylenetetraamine); N, N"-diethyl-N, N', N"-trimethyl(dipropylenetriamine); N, N"-dipropyl-N, N', N"-trimethyl(dipropylenetriamine); N, N"-diisopropyl-N, N', N"-trimethyl(dipropylenetriamine); N, N"-dibutyl-N, N', N"-trimethyl(dipropylenetriamine); N, N"-diisobutyl-N, N', N"-trimethyl(dipropylenetriamine); N, N"-disecbutyl-N, N', N"-trimethyl(dipropylenetriamine); N, N"-ditertbutyl-N, N', N"-trimethyl(dipropylenetriamine); N, N"-dipentyl-N, N', N"-trimethyl(dipropylenetriamine); N, N"-diisopentyl-N, N', N"-trimethyl(dipropylenetriamine); N, N"-ditertpentyl-N, N', N"-trimethyl(dipropylenetriamine); N, N"-dineopentyl-N, N', N"-trimethyl(dipropylenetriamine); N, N"-disecpentyl-N, N', N"-trimethyl(dipropylenetriamine); N, N"-di(3-pentyl)-N, N', N"-trimethyl(dipropylenetriamine); N, N"-disecisoopentyl-N, N', N"-trimethyl(dipropylenetriamine); N, N"-dihexyl-N, N', N"-trimethyl(dipropylenetriamine); N, N"-diisohexyl-N, N', N"-trimethyl(dipropylenetriamine); N, N"-dineohexyl-N, N', N"-trimethyl(dipropylenetriamine); N, N"'-diethyl-N, N', N",N‴-tetramethyl(tripropylenetetraamine); N, N"'-dipropyl-N, N', N",N‴-tetramethyl(tripropylenetetraamine); N, N"'-diisopropyl-N, N', N",N‴-tetramethyl(tripropylenetetraamine); N, N"'-dibutyl-N, N', N",N‴-tetramethyl(tripropylenetetraamine); N, N"'-disobutyl-N, N', N",N‴-tetramethyl(tripropylenetetraamine); N, N"'-disecbutyl-N, N', N",N‴-tetramethyl(tripropylenetetraamine); N, N‴-ditertbutyl-N, N', N",N‴-tetramethyl(tripropylenetetraamine); N, N"'-dipentyl-N, N', N",N‴-tetramethyl(tripropylenetetraamine); N, N‴-diisopentyl-N, N', N",N‴-tetramethyl(tripropylenetetraamine); N, N"'-ditertpentyl-N, N', N",N‴-tetramethyl(tripropylenetetraamine); N, N"'-dineopentyl-N, N', N",N‴-tetramethyl(tripropylenetetraamine); N, N"'-disecpentyl-N, N', N",N‴-tetramethyl(tripropylenetetraamine); N, N"'-di(3-pentyl)-N, N', N",N‴-tetramethyl(tripropylenetetraamine); N, N"'-disecisopentyl-N, N', N",N‴-tetramethyl(tripropylenetetraamine); N, N"'-dihexyl-N, N', N",N‴-tetramethyl(tripropylenetetraamine); N, N"'-diisohexyl-N, N', N",N‴-tetramethyl(tripropylenetetraamine); N, N"'-dineohexyl-N, N', N",N‴-tetramethyl(tripropylenetetraamine); N, N'-diethyl-N, N'-dimethyl-bis(aminoethyl)ether; N, N'-dipropyl-N, N'-dimethyl-bis(aminoethyl)ether; N, N'-diisopropyl-N, N'-dimethyl-bis(aminoethyl)ether; N, N'-dibutyl-N, N'-dimethyl-bis(aminoethyl)ether; N, N'-diisobutyl-N, N'-dimethyl-bis(aminoethyl)ether; N, N'-disecbutyl-N, N'-dimethyl-bis(aminoethyl)ether; N, N'-ditertbutyl-N, N'-dimethyl-bis(aminoethyl)ether; N, N'-dipentyl-N, N'-dimethyl-bis(aminoethyl)ether; N, N'-dibutyl-N, N'-diisopentyl-bis(aminoethyl)ether; N, N'-ditertpentyl-N, N'-dimethyl-bis(aminoethyl)ether; N, N'-dineopentyl-N, N'-dimethyl-bis(aminoethyl)ether; N, N'-disecpentyl-N, N'-dimethyl-bis(aminoethyl)ether; N, N'-di(3-pentyl)-N, N'-dimethyl-bis(aminoethyl)ether; N, N'-disecisoopentyl -N, N'-dimethyl-bis(aminoethyl)ether; N, N'-dihexyl-N, N'-dimethyl-bis(aminoethyl)ether; N, N'-diisohexyl-N, N'-dimethyl-bis(aminoethyl)ether; N, N'-dineohexyl-N, N'-dimethyl-bis(aminoethyl)ether; bis(2-azetidinoethyl)ether; bis(2-pyrrolidinoethyl)ether; bis(2-piperidinodinoethyl)ether; bis(2-azepanoethyl)ether; bis(2-azocanoethyl)ether, and the like, or any combination thereof.

In one embodiment, the catalyst composition according to the invention can be used as the sole catalyst or alternatively in combination with at least one tertiary amine catalyst. The tertiary amine catalyst can have at least one isocyanate reactive group or alternatively it can be a conventional tertiary amine catalyst having no-isocyanate reactive groups. Examples of isocyanate reactive groups comprise a primary hydroxyl group, a secondary hydroxyl group, a primary amine group, a secondary amine group, a urea group or an amide group. Examples of tertiary amine catalysts having an isocyanate reactive group preferably include, but are not limited to N, N-bis(3-dimethylaminopropyl)-N-isopropanolamine; N, N-dimethylaminoethyl-N'-methyl ethanolamine; N, N, N'-trimethylaminopropylethanolamine; N, N-dimethylethanolamine; N, N-diethylethanolamine; N, N-dimethyl-N', N'-2-hydroxy(propyl)-1,3-propylenediamine; dimethylaminopropylamine; (N, N-dimethylaminoethoxy) ethanol; methyl-hydroxy-ethyl-piperazine; bis(N, N-dimethyl-3-aminopropyl) amine; N, N-dimethylaminopropyl urea; diethylaminopropyl urea; N, N'-bis(3-dimethylaminopropyl)urea; N, N'-bis(3-diethylaminopropyl)urea; bis(dimethylamino)-2-propanol; 6-dimethylamio-1-hexanol; N-(3-aminopropyl) imidazole); N-(2-hydroxypropyl) imidazole; N-(2-hydroxyethyl) imidazole; 2-[N-(dimethylaminoethoxyethyl)-N-methylamino] ethanol; N, N-dimethylaminoethyl-N'-methyl-N'-ethanol; dimethylaminoethoxyethanol; N, N, N'-trimethyl-N'-3-aminopropyl-bis(aminoethyl) ether; or a combination thereof. The weight ratio of suitable tertiary amines to the inventive catalyst can preferably range from about 0 to about 100, about 0.1 to about 50 and in some cases about 1 to about 10.

In another embodiment, the tertiary amine catalyst component is highly volatile and is not isocyanate-reactive. For example, in one embodiment, the tertiary amine catalyst component is a volatile gelling catalyst and preferably is or includes diazobicyclooctane (triethylenediamine), 1,8-diazabicycloundec-7-ene, tris(dimethylaminopropyl) amine, dimethylaminocyclohexylamine, bis(dimethylaminopropyl)-N-methylamine, 2, 2'-dimorpholinodiethyl ether, N-methylimidazole, 1,2-dimethylimidazole, 1-ethylimidazole, N-methylmorpholine, N-ethylmorpholine, N-cetylmorpholine or combinations thereof. Additionally or alternatively, in one embodiment, the tertiary amine catalyst component is or includes a volatile blowing catalyst and is or includes bis-dimethylaminoethyl ether, pentamethyldiethylenetriamine, hexamethyltriethylenetetramine, heptamethyltetraethylenepentamine and related compositions, higher permethylated polyamines, 2-[N-(dimethylaminoethoxyethyl)-N-methylamino]ethanol and related structures, alkoxylated polyamines, imidazole-boron compositions, amino propyl-bis(amino-ethyl) ether compositions, or combinations thereof.

In another embodiment, when tertiary amine catalysts according to the invention are used in combination with a secondary tertiary amine catalyst such as 2, 2'-dimorpholinodiethyl ether, N-methylimidazole, 1,2-dimethylimidazole, 1-ethylimidazole and the like, further improvement in the stabilization of hydrohaloolefin premix is observed and it can not be accounted by the simple contribution to the stabilization of each individual tertiary amine catalyst component as shown in example 17 and example 18.

In another embodiment, the inventive catalyst can also preferably be acid blocked with an acid including carboxylic acids (alkyl, substituted alkyl, alkylene, aromatic, substituted aromatic), sulfonic acids or any other organic or inorganic acid. Examples of preferable carboxylic acids include mono-acids, di-acids or poly-acids with or without isocyanate reactive groups. Examples of carboxylic acids include formic acid, acetic acid, propionic acid, butanoic acid, pentanoic acid, neopentanoic acid, hexanoic acid, 2-ethylhexyl carboxylic acid, neohexanoic acid, octanoic acid, neooctanoic acid, heptanoic acid, neoheptanoic acid, nonanoic acid, neononanoic acid, decanoic acid, neodecanoic acid, undecanoic acid, neoundecanoic acid, dodecanoic acid, neododecanoic acid, myristic acid, pentadecanoic acid, hexadecanoic acid, heptadecanoic acid, octadecanoic acid, benzoic acid, oxalic acid, malonic acid, succinic acid, glutaric acid, adipic acid, pimelic acid, suberic acid, azelaic acid, sebacic acid, glycolic acid, lactic acid, tartaric acid, citric acid, malic acid, salicylic acid and the like. An acid blocked catalyst can be obtained by known methods using conventional equipment.

In another embodiment, the tertiary amine catalyst component is preferably used in conjunction with a metal catalyst. For example, in one embodiment, the tertiary amine catalyst component is preferably used with an organotin compound, tin(II) carboxylate salts, bismuth(III) carboxylate salts, or combinations thereof. Examples of preferable transition metal catalysts such as organotin compounds or bismuth carboxylates can comprise at least one member selected from the group consisting of dibutylin dilaureate, dimethyltin dilaureate, dimethyltin diacetate, dibutyltin diacetate, dimethyltin dilaurylmercaptide, dibutyltin dilaurylmercaptide, dimethyltin diisooctylmaleate, dibutyltin diisooctylmaleate, dimethyltin bi(2-ethylhexyl mercaptacetate), dibutyltin bi(2-ethylhexyl mercaptacetate), stannous octate, other suitable organotin catalysts, or a combination thereof. Other metals can also be included, such as, for example, bismuth (Bi). Suitable bismuth carboxylate salts preferably include salts of pentanoic acid, neopentanoic acid, hexanoic acid, 2-ethylhexyl carboxylic acid, neohexanoic acid, octanoic acid, neooctanoic acid, heptanoic acid, neoheptanoic acid, nonanoic acid, neononanoic acid, decanoic acid, neodecanoic acid, undecanoic acid, neoundecanoic acid, dodecanoic acid, neododecanoic acid, and other suitable carboxylic acids. Other metal salts of of lead (Pb), iron (Fe), zinc (Zn) with pentanoic acid, neopentanoic acid, hexanoic acid, 2-ethylhexyl carboxylic acid, octanoic acid, neooctanoic acid, neoheptanoic acid, neodecanoic acid, neoundecanoic acid, neododecanoic acid, and other suitable carboxylic acids may also be included.

The catalyst composition as defined in formula I can be produced, for example, by following these steps; Step 1: reacting a mixture of polyalkylene polyamine with a corresponding ketone in a stainless steel reactor equipped with mechanical stirrer, heating mantle and cooling coil in the presence of 5% Pt/C that are charged into a stainless steel reactor. Next, the steel reactor is sealed and purged with nitrogen gas for three times followed by purging with hydrogen gas for three times while stirring. Then the reactor is heated to 120°C and the pressure of hydrogen gas is set at 800 psi until the hydrogen uptake finished and kept for an additional hour. The reactor is vented after cooling to room temperature. All volatiles are removed on a rotary evaporator under reduced pressure, and the product is collected as a mixture of material containing, in one aspect of the invention, di-alkylated polyalkylene polyamine; Step 2: placing the di-alkylated polyalkylene polyamine in a stainless steel reactor equipped with mechanical stirrer, heating mantle and cooling coil in the presence of 5% Pd/C that are charged into a stainless steel reactor. Formaldehyde 37 wt. % aqueous solution is charged into the high pressure syringe pump. Formaldehyde solution is fed from the pump into the reactor for about 2-3 hours until the methylation is completed. The reactor is vented after cooling to room temperature. All volatiles are removed on rotary evaporator under reduced pressure, and the product is collected as a mixture of material containing, in one aspect of the invention, di-alkylated permethylated polyalkylene polyamine.

In another embodiment, the catalyst system or compositions of the present invention can preferably further comprise other catalytic materials such as carboxylate salts in any amount. Preferably, the other catalytic materials are selected from alkali metal salts, alkaline earth metal salts, and quaternary ammonium carboxylate salts including, but are not limited to, potassium formate, potassium acetate, potassium propionate, potassium butanoate, potassium pentanoate, potassium hexanoate, potassium heptanoate, potassium octoate, potassium 2-ethylhexanoate, potassium decanoate, potassium butyrate, potassium isobutyrate, potassium nonante, potassium stearate, sodium octoate, lithium stearate, sodium caprioate, lithium octoate, 2-hydroxypropyltrimethylammonium octoate solution, and the like, or any combination thereof.

Preferably, the amount of the other catalytic materials and salts can range from about 0 pphp to about 20 pphp, about 0.1 pphp to about 15 pphp and in some cases about 0.5 pphp to about 10 pphp.

It is also within the scope of the catalyst composition of this invention to include mixtures or combinations of more that one catalyst composition according to the invention. Additionally, in another embodiment the catalyst system or the compositions of the present invention can preferably also further comprise at least one urethane catalyst having no isocyanate reactive groups.

The term "contact product" is used herein to describe compositions wherein the components are contacted together in any order, in any manner, and for any length of time. For example, the components can be contacted by blending or mixing. Further, contacting of any component can occur in the presence or absence of any other component of the compositions or foam formulations described herein. Combining additional catalyst components can be done by any method known to one of skill in the art. For example, in one aspect of the present invention, catalyst compositions can be prepared by combining or contacting the catalyst composition as defined in Formula I with at least one tertiary amine having or not at least one isocyanate reactive group and optionally with an alkali metal carboxylate salt. This typically occurs in solution form.

While compositions and methods are described in terms of "comprising" various components or steps, the compositions and methods can also "consist essentially of" or "consist of" the various components or steps.

### POLYISOCYANATES

Polyisocyanates that are useful in the PIR/PUR foam formation process preferably include, but are not limited to, hexamethylene diisocyanate, isophorone diisocyanate, phenylene diisocyante, toluene diisocyanate (TDI), diphenyl methane diisocyanate isomers (MDI), hydrated MDI and 1,5-naphthalene diisocyanate. For example, 2,4-TDI, 2,6-TDI, and mixtures thereof, can be readily employed in the present invention. Other suitable mixtures of diisocyanates include, but are not limited to, those known in the art as crude MDI, or PAPI, which contain 4,4'-diphenylmethane diisocyanate along with other isomeric and analogous higher polyisocyanates. In another aspect of this invention, prepolymers of polyisocyanates comprising a partially prereacted mixture of polyisocyanates and polyether or polyester polyol are suitable. In still another aspect, the polyisocyanate comprises MDI, or consists essentially of MDI or mixtures of MDI's.

The catalyst system, compositions, and methods of producing PIR/PUR foam of the present invention can be used to manufacture many types of foam. This catalyst system is useful, for example, in the formation of foam products for rigid and flame retardant applications, which usually require a high Isocyanate Index. As defined previously, Isocyanate Index is the actual amount of polyisocyanate used divided by the theoretically required stoichiometric amount of polyisocyanate required to react with all the active hydrogen in the reaction mixture, multiplied by 100. For purposes of the present invention, Isocyanate Index is represented by the equation: Isocyanate Index = (Eq NCO/Eq of active hydrogen)x100, wherein Eq NCO is the number of NCO functional groups in the polyisocyanate, and Eq of active hydrogen is the number of equivalent active hydrogen atoms.

Foam products which are produced with an Isocyanate Index from about 10 to about 800 are within the scope of this invention. In accordance with other aspects of the present invention, the Isocyanate Index ranges from about 20 to about 700, from about 30 to about 650, from about 50 to about 600, or from about 70 to about 500.

### POLYOLS

Active hydrogen-containing compounds for use with the foregoing polyisocyanates in forming the polyisocyanurate/polyurethane foams of this invention can be any of those organic compounds having at least two hydroxyl groups such as, for example, polyols. Polyols that are typically used in PIR/PUR foam formation processes include polyalkylene ether and polyester polyols. The polyalkylene ether polyol includes the poly(alkyleneoxide) polymers such as poly(ethyleneoxide) and poly(propyleneoxide) polymers and copolymers with terminal hydroxyl groups derived from polyhydric compounds, including diols and triols. These preferably include, but are not limited to, ethylene glycol, propylene glycol, 1,3-butane diol, 1,4-butane diol, 1,6-hexane diol, neopentyl glycol, diethylene glycol, dipropylene glycol, pentaerythritol, glycerol, diglycerol, trimethylol propane, cyclohexane diol, and sugars such as sucrose and like low molecular weight polyols.

Amine polyether polyols can be used in the present invention. These can be prepared when an amine such as, for example, ethylenediamine, diethylenetriamine, tolylenediamine, diphenylmethanediamine, or triethanolamine is reacted with ethylene oxide or propylene oxide.

In another aspect of the present invention, a single high molecular weight polyether polyol, or a mixture of high molecular weight polyether polyols, such as mixtures of different multifunctional materials and/or different molecular weight or different chemical composition materials can be used.

In yet another aspect of the present invention, polyester polyols can be used, including those produced when a dicarboxylic acid is reacted with an excess of a diol. Non-limiting examples include adipic acid or phathalic acid or phthalic anhydride reacting with ethylene glycol or butanediol. Polyols useful in the present invention can be produced by reacting a lactone with an excess of a diol, for example, caprolactone reacted with propylene glycol. In a further aspect, active hydrogen-containing compounds such as polyester polyols and polyether polyols, and combinations thereof, are useful in the present invention.

The polyol can have an OH number of about 5 to about 600, about 100 to about 600 and in some cases about 50 to about 100.and a functionality of about 2 to about 8, about 3 to about 6 and in some cases about 4 to about 6.

The amount of each type of polyol can range from about 0 pphp to about 100 pphp about 10 pphp to about 90 pphp and in some cases about 20 pphp to about 80 pphp.

### BLOWING AGENTS

In accordance with the compositions, foam formulations, and methods of producing PIR/PUR foam within the scope of the present invention, suitable blowing agents that can be used alone or in combination preferably include, but are not limited to, water, methylene chloride, acetone, hydrofluorocarbons (HFCs), hydrochlorocarbons (HCCs), hydrofluoroolefins (HFOs), chlorofluoroolefins (CFOs), hydrochloroolefins (HCOs), hydrofluorochloroolefins (HFCOs), hydrochlorofluorocarbons (HCFCs), chloroolefins, formates and hydrocarbons. Examples of HFCs include, but are not limited to, HFC-245fa, HFC-134a, and HFC-365; illustrative examples of HCFCs include, but are not limited to, HCFC-141b, HCFC-22, and HCFC-123. Exemplary hydrocarbons include, but are not limited to, n-pentane, iso-pentane, cyclopentane, and the like, or any combination thereof. In one aspect of the present invention, the blowing agent or mixture of blowing agents comprises at least one hydrocarbon. In another aspect, the blowing agent comprises n-pentane. Yet, in another aspect of the present invention, the blowing agent consists essentially of n-pentane or mixtures of n-pentane with one or more blowing agents. Examples of hydrohaloolefin blowing agents are HFO-1234ze (trans-1,3,3,3-Tetrafluoroprop-1-ene), HFO-1234yf (2,3,3,3-Tetrafluoropropene) and HFCO-1233zd (1-Propene,1-chloro-3,3,3-trifluoro), among other HFOs.

The blowing agent component comprises a hydrohaloolefin, preferably comprising at least one of trans-HFO-1234ze and HFCO-1233zd., and optionally a hydrocarbon, fluorocarbon, chlorocarbon, fluorochlorocarbon, halogenated hydrocarbon, ether, fluorinated ether, ester, aldehyde, ketone, carbon dioxide generating material, or combinations thereof. The hydrohaloolefin preferably comprises at least one halooalkene such as a fluoroalkene or chloroalkene containing from 3 to 4 carbon atoms and at least one carbon-carbon double bond. Preferred hydrohaloolefins non-exclusively include trifluoropropenes, tetrafluoropropenes such as (HFO-1234), pentafluoropropenes such as (HFO-1225), chlorotrifloropropenes such as (HFO-1233), chlorodifluoro propenes, chlorotrifluoropropenes, chlorotetrafluoropropenes, and combinations of these. Other preferred blowing agents comprise the tetrafluoropropene, pentafluoropropene, and chlorotrifloropropene compounds in which the unsaturated terminal carbon has not more than one fluorine or chlorine substituent. Included are 1,3,3,3- tetrafluoropropene (HFO-1234ze); 1,1,3,3-tetrafluoropropene; 1,2,3,3,3-pentafluoropropene (HFO-1225ye); 1,1,1-trifluoropropene; 1,1,1,3,3-pentafluoropropene (HFO 1225zc); 1,1,1,3,3,3-hexafluorobut-2-ene, 1,1,2,3,3- pentafluoropropene (HFO-1225yc); 1,1,1,2,3- pentafluoropropene (HFO-1225yez); 1-chloro-3,3,3-trifluoropropene (HFCO-1233zd); 1,1,1,4,4,4-hexafluorobut-2-ene or combinations thereof, and any and all structural isomers, geometric isomers, or stereoisomers of each of these. Preferred optional blowing agents non-exclusively include water, formic acid, organic acids that produce carbon dioxide when they react with an isocyanate, hydrocarbons; ethers, halogenated ethers; pentafluorobutane; pentafluoropropane; hexafluoropropane; heptafluoropropane; trans-1,2 dichloro-ethylene; methyl formate; 1-chloro-1,2,2,2-tetrafluoroethane; 1,1-dichloro-1-fluoroethane; 1,1,1,2-tetrafluoroethane; 1,1,2,2-tetrafluoroethane; 1-chloro-1,1-difluoroethane; 1,1,1,3,3-pentafluorobutane; 1,1,1,2,3,3,3-heptafluoropropane; trichlorofluoromethane; dichlorodifluoromethane; 1,1,1,3,3, 3-hexafluoropropane; 1,1,1,2,3,3-hexafluoropropane; difluoromethane; difluoroethane; 1,1,1,3,3-pentafluoropropane; 1,1-difluoroethane; isobutane; normal pentane; isopentane; cyclopentane, or combinations thereof. The blowing agent component is usually present in the polyol premix composition in an amount of from about 1 wt.% to about 30 wt.%, preferably from about 3 wt.% to about 25 wt.%, and more preferably from about 5 wt.% to about 25 wt.%, by weight of the polyol premix composition. When both a hydrohaloolefin and an optional blowing agent are present, the hydrohaloolefin component is usually present in the blowing agent component in an amount of from about 5 wt.% to about 90 wt.%, preferably from about 7 wt.% to about 80 wt.%, and more preferably from about 10 wt.% to about 70 wt.%, by weight of the blowing agent component; and the optional blowing agent is usually present in the blowing agent component in an amount of from about 95 wt. % to about 10 wt.%, preferably from about 93 wt.% to about 20 wt.%, and more preferably from about 90 wt.% to about 30 wt.%, by weight of the blowing agent component.

Due to the discovery that chlorofluorocarbons (CFCs) can deplete ozone in the stratosphere, this class of blowing agents is not desirable for use. A chlorofluorocarbon (CFC) is an alkane in which all hydrogen atoms are substituted with chlorine and fluorine atoms. Examples of CFCs include trichlorofluoromethane and dichlorodifluoromethane.

The amount of blowing agent used can vary based on, for example, the intended use and application of the foam product and the desired foam stiffness and density. In the compositions, foam formulations and methods for preparing a polyisocyanurate/polyurethane foam of the present invention, the blowing agent is present in amounts from about 5 to about 80 parts by weight per hundred weight parts of the at least one active hydrogen-containing compound. In another aspect, the blowing agent is present in amounts from about 10 to about 60, from about 15 to about 50, or from about 20 to about 40, parts by weight per hundred weight parts of the at least one active hydrogen-containing compound. If the at least one active hydrogen-containing compound is an at least one polyol, the blowing agent is present in amounts from about 5 to about 80 parts by weight per hundred weight parts polyol (pphp), from about 10 to about 60 pphp, from about 15 to about 50 pphp, or from about 20 to about 40 pphp.

If water is present in the formulation, for use as a blowing agent or otherwise, water is present in amounts up to about 60 parts by weight per hundred weight parts of the at least one active hydrogen-containing compound. Likewise, if the at least one active hydrogen-containing compound is an at least one polyol, water can range from 0 to about 15 pphp. In another aspect, water can range from 0 to about 10 pphp, from 0 to about 8 pphp, from 0 to about 6 pphp, or from 0 to about 4 pphp.

### URETHANE CATALYST

In one embodiment, conventional urethane catalysts having no isocyanate reactive group can preferably be employed to accelerate the reaction to form polyurethanes, and can be used as a further component of the catalyst systems and compositions of the present invention to produce polyisocyanurate/polyurethane foam. Urethane catalysts suitable for use herein preferably include, but are not limited to, metal salt catalysts, such as organotins, and amine compounds, such as triethylenediamine (TEDA), N-methylimidazole, 1,2-dimethyl-imidazole, N-methylmorpholine (commercially available as the DABCO^{®} NMM catalyst), N-ethylmorpholine (commercially available as the DABCO^{®} NEM catalyst), triethylamine (commercially available as the DABCO^{®} TETN catalyst), N,N'-dimethylpiperazine, 1,3,5-tris(dimethylaminopropyl)hexahydrotriazine (commercially available as the Polycat^{®} 41 catalyst), 2,4,6-tris(dimethylaminomethyl)phenol (commercially available as the DABCO TMR^{®} 30 catalyst), N-methyldicyclohexylamine (commercially available as the Polycat^{®} 12 catalyst), pentamethyldipropylene triamine (commercially available as the Polycat^{®} 77 catalyst), N-methyl-N'-(2-dimethylamino)-ethyl-piperazine, tributylamine, pentamethyldiethylenetriamine (commercially available as the Polycat^{®} 5 catalyst), hexamethyltriethylenetetramine, heptamethyltetraethylenepentamine, dimethylaminocyclohexylamine (commercially available as the Polycat^{®} 8 catalyst), pentamethyldipropylene-triamine, triethanolamine, dimethylethanolamine, bis(dimethylaminoethyl)ether (commercially available as the DABCO^{®} BL19 catalyst), tris(3-dimethylamino)-propylamine (commercially available as the Polycat^{®} 9 catalyst), 1,8-diazabicyclo[5.4.0] undecene (commercially available as the DABCO^{®} DBU catalyst) or its acid blocked derivatives, and the like, as well as any mixture thereof.

In another embodiment, the present invention can be used with tertiary amine catalysts having isocyanate reactive groups. Preferably, the isocyanate reactive groups present in the tertiary amine gelling co-catalyst consist essentially of primary amine, secondary amine, secondary-hydroxyl group, amide and urea. Examples of gelling catalysts preferably include N,N-bis(3-dimethylamino-propyl)-N-(2-hydroxypropyl) amine; N,N-dimethyl-N',N'-bis(2-hydroxypropyl)-1,3-propylenediamine; dimethylaminopropylamine (DMAPA); N-methyl-N-2-hydroxypropyl-piperazine, bis(dimethylaminopropyl)amine (POLYCAT^{®} 15), dimethylaminopropylurea and N,N'-bis(3-dimethylaminopropyl) urea (DABCO^{®} NE1060, DABCO^{®} NE1070, DABCO^{®} NE1080 and DABCO^{®} NE1082), 1,3-bis(dimethylamino)-2-propanol, 6-dimethylamino-1-hexanol, N-(3-aminopropyl)imidazole, N-(2-hydroxypropyl)imidazole, N,N'-bis(2-hydroxypropyl) piperazine, N-(2-hydroxypropyl)-morpholine, N-(2-hydroxyethylimidazole). Examples of blowing co-catalysts containing isocyanate reactive groups that can be used with the above mentioned gelling catalysts preferably include 2-[N-(dimethylaminoethoxyethyl)-N-methylamino]ethanol (DABCO^{®} NE200), N,N,N'-trimethyl-N'-3-aminopropyl-bis(aminoethyl) ether (DABCO^{®} NE300).

Suitable urethane catalysts that can be used in combination with the inventive catalyst preferably also include acid blocked tertiary amines with acids including carboxylic acids (alkyl, substituted alkyl, alkylene, aromatic, substituted aromatic), sulfonic acids or any other organic or inorganic acid. Examples of carboxylic acids preferably include mono-acids, di-acids or poly-acids with or without isocyanate reactive groups. Examples of carboxylic acids include formic acid, acetic acid, propionic acid, butanoic acid, pentanoic acid, neopentanoic acid, hexanoic acid, 2-ethylhexyl carboxylic acid, neohexanoic acid, octanoic acid, neooctanoic acid, heptanoic acid, neoheptanoic acid, nonanoic acid, neononanoic acid, decanoic acid, neodecanoic acid, undecanoic acid, neoundecanoic acid, dodecanoic acid, neododecanoic acid, myristic acid, pentadecanoic acid, hexadecanoic acid, heptadecanoic acid, octadecanoic acid, benzoic acid, oxalic acid, malonic acid, succinic acid, glutaric acid, adipic acid, pimelic acid, suberic acid, azelaic acid, sebacic acid, glycolic acid, lactic acid, tartaric acid, citric acid, malic acid, salicylic acid and the like. An acid blocked catalyst can be obtained by known methods using conventional equipment.

In another embodiment, the tertiary amine catalyst component can also be used in conjunction with a metal catalyst. For example, in one embodiment, the tertiary amine catalyst component is preferably used with an organotin compound, tin(ll) carboxylate salts, bismuth(lll) carboxylate salts, or combinations thereof. Preferable examples of transition metal catalysts such as organotin compounds or bismuth carboxylates can comprise at least one member selected from the group consisting of dibutylin dilaureate, dimethyltin dilaureate, dimethyltin diacetate, dibutyltin diacetate, dimethyltin dilaurylmercaptide, dibutyltin dilaurylmercaptide, dimethyltin diisooctylmaleate, dibutyltin diisooctylmaleate, dimethyltin bi(2-ethylhexyl mercaptacetate), dibutyltin bi(2-ethylhexyl mercaptacetate), stannous octate, other suitable organotin catalysts, or a combination thereof. Other metals can also be included, such as, for example, bismuth (Bi). Suitable bismuth carboxylate salts preferably include salts of pentanoic acid, neopentanoic acid, hexanoic acid, 2-ethylhexyl carboxylic acid, neohexanoic acid, octanoic acid, neooctanoic acid, heptanoic acid, neoheptanoic acid, nonanoic acid, neononanoic acid, decanoic acid, neodecanoic acid, undecanoic acid, neoundecanoic acid, dodecanoic acid, neododecanoic acid, and other suitable carboxylic acids. Other salts of transition metals of lead (Pb), iron (Fe), zinc (Zn) with pentanoic acid, neopentanoic acid, hexanoic acid, 2-ethylhexyl carboxylic acid, octanoic acid, neooctanoic acid, neoheptanoic acid, neodecanoic acid, neoundecanoic acid, neododecanoic acid, and other suitable carboxylic acids may also be included.

In another embodiment, the present invention can preferably further comprise other catalytic materials such as carboxylate salts in any amount. Preferable examples of alkali metal, alkaline earth metal, and quaternary ammonium carboxylate salts include, but are not limited to, potassium formate, potassium acetate, potassium propionate, potassium butanoate, potassium pentanoate, potassium hexanoate, potassium heptanoate, potassium octoate, potassium 2-ethylhexanoate, potassium decanoate, potassium butyrate, potassium isobutyrate, potassium nonante, potassium stearate, sodium octoate, lithium stearate, sodium caprioate, lithium octoate, 2-hydroxypropyltrimethylammonium octoate solution, tetramethylammonium carboxylates, tetralkylammonium carboxylates such as tetramethylammonium pivalate (supplied by Evonik Corporation as DABCO^{®}TMR7) and the like, or any combination thereof.

For preparing a polyisocyanurate/polyurethane foam of the present invention, the urethane catalyst can be present in the formulation from 0 to about 10 pphp, from 0 to about 8 pphp, from 0 to about 6 pphp, from 0 to about 4 pphp, from 0 to about 2 pphp, or from 0 to about 1 pphp. In another aspect, the urethane catalyst is present from 0 to about 0.8 pphp, from 0 to about 0.6 pphp, from 0 to about 0.4 pphp, or from 0 to about 0.2 pphp.

### MISCELLANEOUS ADDITIVES

Depending on the requirements during foam manufacturing or for the end-use application of the foam product, various additives can preferably be employed in the PIR/PUR foam formulation to tailor specific properties. These additives preferably include, but are not limited to, cell stabilizers, flame retardants, chain extenders, epoxy resins, acrylic resins, fillers, pigments, or any combination thereof. It is understood that other mixtures or materials that are known in the art can be included in the foam formulations and are within the scope of the present invention.

Cell stabilizers include surfactants such as organopolysiloxanes. Silicon surfactants can be present in the foam formulation in amounts from about 0.5 to about 10 pphp, about 0.6 to about 9 pphp, about 0.7 to about 8 pphp, about 0.8 to about 7 pphp, about 0.9 to about 6 pphp, about 1 to about 5 pphp, or about 1.1 to about 4 pphp. Useful flame retardants include halogenated organophosphorous compounds and non-halogenated compounds. A non-limiting example of a halogenated flame retardant is trichloropropylphosphate (TCPP). For example, triethylphosphate ester (TEP) and DMMP are non-halogenated flame retardants. Depending on the end-use foam application, flame retardants can be present in the foam formulation in amounts from 0 to about 50 pphp, from 0 to about 40 pphp, from 0 to about 30 pphp, or from 0 to about 20 pphp. In another aspect, the flame retardant is present from 0 to about 15 pphp, 0 to about 10 pphp, 0 to about 7 pphp, or 0 to about 5 pphp. Chain extenders such as ethylene glycol and butane diol can also be employed in the present invention. Ethylene glycol, for instance, can also be present in the formulation as a diluent or solvent for the carboxylate salt catalysts of the present invention.

### POLYURETHANE FOAM FORMULATION AND PROCESS

One aspect of the present invention provides for a composition comprising the contact product of at least one active hydrogen-containing compound, at least one blowing agent, and at least one catalyst composition according to the invention.

Another aspect provides a composition comprising the contact product of at least one polyisocyanate, at least one blowing agent, and at least one inventive catalyst composition as defined above used in combination with at least one tertiary amine having at least one isocyanate reactive group.

Another aspect provides a composition comprising the contact product of at least one polyisocyanate, at least one blowing agent, and at least one inventive catalyst composition used in combination with at least one tertiary amine having no isocyanate reactive group.

In another embodiment, the composition can preferably further comprise the inventive catalyst composition as defined above with at least one urethane catalyst having no isocyanate reactive group and at least one urethane catalyst having an isocyanate reactive group.

In another embodiment, the composition can preferably further comprise at least one additive selected from at least one cell stabilizer, at least one flame retardant, at least one chain extender, at least one epoxy resin, at least one acrylic resin, at least one filler, at least one pigment, or any combination thereof.

The present invention provides a method for preparing a polyurethane foam as well as a polyisocyanurate/polyurethane (PIR/PUR) foam which comprises contacting at least one polyisocyanate with at least one active hydrogen-containing compound, in the presence of at least one blowing agent and an effective amount of inventive catalyst composition as defined above. In accordance with the method of the present invention, PUR as well as PIR/PUR foams can be produced having a density from about 16 Kg/m³ to about 250 Kg/m³ (about 0.5 lb/ft³ to about 15.5 lb/ft³), or from about 24 Kg/m³ to about 60 Kg/m³ (about 1.5 lb/ft³ to about 3.75 lb/ft³).

The instant invention can be used in a wide range of methods for making any kind of rigid closed-cell foams as well as rigid open cell foams. Examples of suitable methods comprise molding, spraying, among other rigid foam production methods. In one aspect the inventive method relates to a method for making a laminated foam, appliance foam, rigid closed-cell foam for commercial refrigeration and disco panel. The inventive foam can be laminated to a wide range of substrates including wood, steel, paper and plastic.

The method for preparing PUR as well as PIR/PUR foams also can provide longer polyol premix shelf life when compared to other commercially available catalyst systems, such that the PUR as well as the PIR/PUR foam has improved catalytic activity after ageing in comparison with common industry standards that uses Polycat^{®}-5 as the main catalyst.

The catalyst composition as defined above in formula I should be present in the foam formulation in a catalytically effective amount. In PUR as well as in PIR/PUR foam formulations of the present invention, the catalyst composition is present in amounts from about 0.05 to about 20 parts by weight per hundred weight parts of the at least one active hydrogen-containing compound, excluding the weight contribution of the catalyst system diluent. In another aspect, the catalyst composition is present in amounts from about 0.4 to about 10 parts by weight per hundred weight parts of the at least one active hydrogen-containing compound, or from about 0.8 to about 8 parts by weight per hundred weight parts of the at least one active hydrogen-containing compound. If the at least one active hydrogen-containing compound is an at least one polyol, the catalyst composition is present in amounts from about 0.05 to about 10 parts by weight per hundred weight parts polyol (pphp). In another aspect, the catalyst composition is present in amounts from about 0.2 to about 9.5 pphp, about 0.4 to about 9 pphp, about 0.6 to about 8.5 pphp, or about 0.8 to about 8 pphp.

In accordance with one aspect of the method of the present invention, the components of the foam formulation are contacted substantially contemporaneously. For example, at least one polyisocyanate, at least one active hydrogen-containing compound, at least one blowing agent and an effective amount of catalyst composition according to the invention, are contacted together. Given the number of components involved in PUR and PIR/PUR formulations, there are many different orders of combining the components, and one of skill in the art would realize that varying the order of addition of the components falls within the scope of the present invention. As well, for each of the different orders of combining the aforementioned components of the foam formulation, the foam formulation of the present invention can further comprise at least one urethane catalyst. In addition, the method of producing PIR/PUR foams can preferably further comprise the presence of at least one additive selected from at least one cell stabilizer, at least one flame retardant, at least one chain extender, at least one epoxy resin, at least one acrylic resin, at least one filler, at least one pigment, or any combination thereof. In one aspect of the present invention, all of the components, including optional components, are contacted substantially contemporaneously.

In another aspect of the present invention, a premix of ingredients other than the at least one polyisocyanate are contacted first, followed by the addition of the at least one polyisocyanate. For example, the at least one active hydrogen-containing compound, the at least one blowing agent, and the catalyst composition of the present invention are contacted initially to form a premix. The premix is then contacted with the at least one polyisocyanate to produce PUR or PIR/PUR foams in accordance with the method of the present invention. In a further aspect of the present invention, the same method can be employed, wherein the premix preferably further comprises at least one urethane catalyst. Likewise, the premix can preferably further comprise at least one additive selected from at least one cell stabilizer, at least one flame retardant, at least one chain extender, at least one epoxy resin, at least one acrylic resin, at least one filler, at least one pigment, or any combination thereof.

One aspect of the present invention provides a method for preparing a polyisocyanurate/polyurethane foam comprising:(a) forming a premix comprising:
i) at least one polyol;
ii) about 5 to about 80 parts by weight per hundred weight parts of the polyol (pphp) blowing agent;
iii) about 0.5 to about 10 pphp silicon surfactant;
iv) zero to about 60 pphp water;
v) zero to about 50 pphp flame retardant;
vi) zero to about 10 pphp urethane catalyst; and
vii) about 0.05 to about 20 pphp of a catalyst composition according to the invention as defined above; and
(b) contacting the premix with at least one polyisocyanate at an Isocyanate Index from about 10 to about 800.

### EXAMPLES

These Examples are provided to demonstrate certain aspects of the invention and shall not limit the scope of the claims appended hereto.

Example 1 through 9 describe the syntheses and compositions of seven catalysts and two intermediates formed in accordance with the instant invention.

### EXAMPLE 1 (Comparative)

*This example describes the synthesis of N-isopropyldiethylenetriamine as an intermediate to prepare some catalysts of this invention.*

Procedure: A 2 liter stainless steel reactor was charged with 150 g of DETA (diethylenetriamine) and 2.4 g of 5% Pt/C catalyst (50 % suspension in water) and the reactor was purged with nitrogen. The reactor cooling system was turned on and 107 ml acetone was fed into the reactor at an addition speed sufficient to maintain the reactor at a temperature of less than 35°C. Once feeding was completed the mixture was held at room temperature with mechanical mixing for 1 hour followed by purging the reactor with hydrogen and keeping 800 psig operating pressure.The reactor temperature was slowly increased to keep the hydrogen consumption at about 1 % total expected consumption per minute with a maximum temperature of 120°C. When hydrogen consumption was completed (~ 4.0 hours) the reactor was cooled down to room temperature and the Pt/C catalysts was filtered off from the reactor contents. After rotary evaporation of solvents 187 g of N-isopropyldiethylenetriamine was obtained. Reductive alkylation was carried out by charging a 2 liter stainless steel reactor with 93 g of this product and 135 g of isopropanol with 2.0 g dry weight of 5% Pt(S)/C and the reactor was purged with nitrogen followed by purging with hydrogen. The reactor was heated up to 120°C follow by pressurizing it with hydrogen up to 800 psig. Formalin solution 210.3 ml (37 % formaldehyde in methanol and water) was fed into the reactor over a period of ~1.5 hours until the hydrogen consumption was completed. The reactor was cooled to room temperature and the contents were filtered to remove the Pt(S)/C catalyst. The crude filtered product was transferred to a rotary evaporator to strip off water and methanol yielding 115.6 g of N-isopropyl-N, N', N", N"-tetramethyldiethylenetriamine 54 % based on GC anlaysis.
N-isopropyl-N, N', N", N"-tetramethyldiethylenetriamine is

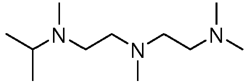

### EXAMPLE 2 (Non-Inventive)

*This example describes the synthesis of N, N"-diisopropyldiethylenetriamine as an intermediate to prepare some catalysts of this invention as well as a comparative catalyst.*

Procedure: A 2 liter stainless steel reactor was charged with 150 g of DETA (diethylenetriamine) and 3.6 g of 5% Pt/C catalyst (50 % suspension in water) and the reactor was purged with nitrogen. The reactor cooling system was turned on and 213.3 g acetone was fed into the reactor at an addition speed sufficient to maintain the reactor at a temperature of less than 31°C. Once feeding was completed the mixture was held at 25°C with mechanical mixing for 1 hour followed by purging the reactor with hydrogen and keeping 800 psig operating pressure.The reactor temperature was initially increased to 50°C then slowly increased to keep the hydrogen consumption at about 1 % total expected consumption per minute with a maximum temperature of 85°C. When hydrogen consumption was completed (~ 3.0 hours) the reactor was cooled down to room temperature and the Pt(S)/C catalyst was filtered off from the reactor contents. The crude filetered product was transferred to a rotary evaporator to strip off methanol and water yielding 234.7 g of product containing N, N"-diisopropyldiethylenetriamine.
N, N"-diisopropyldiethylenetriamine is

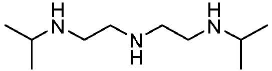

### EXAMPLE 3 (Non-Inventive)

*This example describes the synthesis of N, N', N"-triisopropyldiethylenetriamine as an intermediate to prepare some catalysts of this invention as well as a comparative catalyst.*

Procedure: Procedure: A 2 liter stainless steel reactor was charged with 300 g of DETA (diethylenetriamine) and 20.6 g of 5% Pt(S)/C catalyst (dry weight) and the reactor was purged with nitrogen. The reactor cooling system was turned on and 384.5 mL acetone was fed into the reactor at an addition speed sufficient to maintain the reactor at a temperature of less than 40°C. Once feeding was completed the mixture was held at room temperature with mechanical mixing for 1 hour followed by purging the reactor with hydrogen and keeping 800 psig operating pressure.The reactor temperature was increased to 120°C, and an additional 684 mL acetone was delivered over 3.5 hours. When hydrogen consumption was completed the reactor was cooled down to room temperature and the Pt(S)/C catalyst was filtered off from the reactor contents. After rotary evaporation of solvents, 611 g of N, N', N"-triisopropydiethylenetriamine 71 % based on GC was obtained.
N, N', N"-triisopropyldiethylenetriamine is

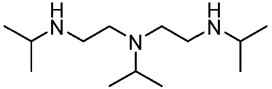

### EXAMPLE 4 (Non-Inventive)

*This example describes the synthesis of N,N"-diisopropyl-N,N',N"-trimethyldiethylenetriamine* as a *catalyst of this invention.*

Procedure 1: A 2 liter stainless steel reactor was charged with 400 g of N, N"-diisopropyldiethylenetriamine from Example 2 and 8 g of 5% Pt(S)/C catalyst (dry weight) and the reactor was purged with nitrogen followed by purging with hydrogen. Temperature was increased to 120°C followed by increasing the pressure to 800 psig H₂. Formalin solution 463 mL was fed into the reactor over a period of ~2 hours until the hydrogen consumption was completed. The reactor was held for an additional 1 hour, then cooled to room temperature. The reactor contents were filtered to remove the Pt(S)/C catalyst. The crude filtered product was transferred to a rotary evaporator to strip off water and methanol yielding 475 g of N,N"-diisopropyl-N,N',N"-trimethyldiethylenetriamine 59 % based on GC analysis.

Procedure 2: A 1 liter stainless steel reactor was charged with 162 g of DETA (diethylenetriamine) and 4.8 g of 5% Pt/C catalyst (50 % suspension in water) and the reactor was purged with nitrogen. The reactor cooling system was turned on and 233.1 mL acetone was fed into the reactor at an addition speed sufficient to maintain the reactor at a temperature of less than 21°C. Once feeding was completed the mixture was held at room temperature with mechanical mixing for 1 hour followed by purging the reactor with hydrogen and keeping 800 psig operating pressure.The reactor temperature was slowly increased to keep the hydrogen consumption at about 1 % total expected consumption per minute with a maximum temperature of 85°C. When hydrogen consumption was completed (~ 2.5 hours) the reactor was cooled down to room temperature and the Pt/C catalysts were filtered off from the reactor contents yielding 332.9 g of N, N"-diisopropyldiethylenetriamine. Reductive alkylation was carried out by transferring the 332.9 g of N, N"-diisopropyldiethylenetriamine and 10.1 g 5% Pd/C into the 2 L stainless steel reactor. The reactor was purged with nitrogen followed by hydrogen purge. The reactor was heated up to 100°C followed by pressurizing it with hydrogen up to 800 psig. The reactor was then fed with 340 mL formalin solution (37 % formaldehyde in methanol and water) over a period of ~4 hours until the hydrogen consumption was completed. The reactor was cooled to room temperature and the contents filtered to remove the Pd/C catalyst. The crude filtered product was transferred to a rotary evaporator to strip off water and methanol yielding N,N"-diisopropyl-N,N',N"-trimethyldiethylenetriamine, 93.4 % based on GC anlaysis.

N,N"-diisopropyl-N,N',N"-trimethyldiethylenetriamine is

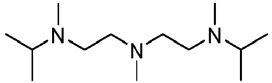

### EXAMPLE 5 (Non-Inventive)

*This example describes the synthesis of N, N', N"-triisopropyl-N, N"-dimethyldiethylenetriamine as an inventive catalyst of this invention.*

Procedure: A 2 liter stainless steel reactor was charged with 80 g of N, N', N"-triisopropyldiethylenetriamine from example 3, 134 g of 2-propanol, 3 g of 5% Pt(S)/C catalyst (dry weight) and the reactor was purged with nitrogen followed by purging with hydrogen. Temperature was increased to 120°C followed by increasing the pressure to 800 psig H₂. Formalin solution 50 g was fed into the reactor over a period of ~1 hour until the hydrogen consumption was completed. The reactor was cooled to room temperature and the contents filtered to remove the Pt(S)/C catalyst. The crude filtered product was transferred to a rotary evaporator to strip off water and methanol yielding 73 g of N, N', N"-triisopropyl-N, N"-dimethyldiethylenetriamine, 74 % based on GC analysis.

N, N', N"-triisopropyl-N, N"-dimethyldiethylenetriamine is

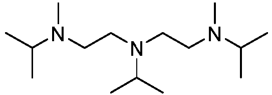

### EXAMPLE 6 (Comparative)

*This example describes the synthesis of N, N"-bis(2-hydroxypropyl)-N, N', N"-triisopropyldiethylenetriamine as a comparative catalyst.*

Procedure: A 300 cc stainless steel reactor was charged with 80 g of N,N',N"-triisopropyldiethylenetriamine from example 3 and the reactor was purged with nitrogen. The temperature of the reactor was increased to 150°C followed by addition of 42 ml of propylene oxide into the reactor over a period of two hours. Once the feeding was completed, the reactor was held at temperature with mechanical mixing for 10 hours. Afterwards, the reactor was cooled down and purged with nitrogen. The reactor contents were placed in a vacuum oven overnight at 60°C under house vacuum to remove any trace of unreacted propylene oxide yielding 108 g of N, N"-bis(2-hydroxypropyl)-N, N', N"-triisopropyldiethylenetriamine.
N, N"-bis(2-hydroxypropyl)-N, N', N"-triisopropyldiethylenetriamine is

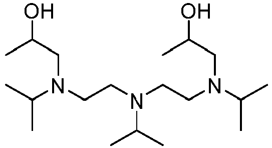

### EXAMPLE 7 (Comparative)

*This example describes the synthesis of N, N' N"-tris(2-hydroxypropyl)-N, N"-diisopropyldiethylenetriamine as a comparative catalyst.*

Procedure: A 300 cc stainless steel reactor was charged with 80 g of N,N"-diisopropyldiethylenetriamine from example 2 and the reactor was purged with nitrogen. The temperature of the reactor was increased to 150°C followed by semi-batch feeding of 88 ml of propylene oxide over 3.5 hours. Once the feeding was completed, the reactor was held at temperature with mechanical mixing for 10 hours. The reactor was then cooled down and purged with nitrogen. The reactor contents were then placed in a vacuum oven overnight at 60°C under vacuum to remove any trace of unreacted propylene oxide yielding 140 g of N, N' N"-tris(2-hydroxypropyl)-N, N"-diisopropyldiethylenetriamine.
N, N' N"-tris(2-hydroxypropyl)-N, N"-diisopropyldiethylenetriamine is

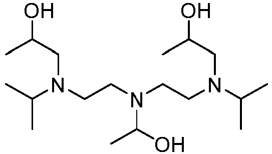

### EXAMPLE 8 (Comparative)

*This example describes the synthesis of N,N"-diisopropyl-N,N',N"-triethyldiethylenetriamine as a catalyst of this invention.*

Procedure: A 2 liter stainless steel reactor was charged with 150 g of N, N"-diisopropylamine from Example 2, 155 g of 2-propanol and 5.1 g of 5% Pt(S)/C catalyst (dry weight) and the reactor was purged with nitrogen followed by purging with hydrogen. Temperature was increased to 120°C followed by increasing the pressure to 800 psig H₂. Acetaldehyde was diluted to 38 % in water and 400 ml of this solution was fed into the reactor over a period of ~3 hours until the hydrogen consumption was completed. The reactor was held at 120°C for 4 hours followed by being cooled to room temperature and the contents filtered to remove the Pt(S)/C catalyst. The crude filtered product was transferred to a rotary evaporator to strip off water and methanol yielding 189 g N,N"-diisopropyl-N, N', N"-triethyldiethylenetriamine.
N,N"-diisopropyl-N,N',N"-triethyldiethylenetriamine is

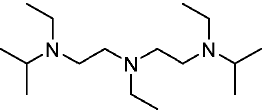

### EXAMPLE 9 (Non-Inventive)

*This example describes the synthesis of N,N"-diisopropyl-N,N',N"-trimethyldipropylenetriamine as a catalyst of this invention.*

Procedure: A 2 liter stainless steel reactor was charged with 208 g N,N-bis(3-aminopropyl)-N-methylamine, 204 g of tetrahydrofuran and 7.9 g of 5% Pt/C catalyst (50 % suspension in water) and the reactor was purged with nitrogen. The reactor temperature was increased to 120°C followed by an increase in hydrogen pressure to 800 psig. Acetone (213 ml) was fed into the reactor over a period of 3.5 hours followed by keeping the temperature at 120°C for about 2 hours. The reactor temperature was then increased to 130°C before feeding 247 ml of formalin solution over a period of ~4 hours until the hydrogen consumption was completed. The reactor was kept at temperature for an additional 2 hours and then cooled to room temperature and the contents filtered to remove the Pt/C catalyst. The crude filtered product was transferred to a rotary evaporator yielding 332.3 g of N,N"-diisopropyl-N,N'N"-trimethyldipropylenetriamine with a 97 % purity based on GC analysis.
N,N"-diisopropyl-N,N',N"-trimethyldipropylenetriamine is

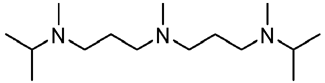

### EXAMPLE 10 (Non-Inventive)

*This example describes the performance of N,N"-diisopropyl-N,N',N"-trimethyldiethylenetriamine made in example 4 in polyurethane systems for spray foams having HFO blowing agents*

The performance and the shelf life stability of N,N"-diisopropyl-N,N',N"-trimethyldiethylenetriamine was carried out using the foam formulation in table 1 (see below). The evaluation of the catalyst reactivity in a spray polyurethane system was conducted using free-rise cup foam sample with a FOAMAT sonar Rate-Of-Rise (ROR) device. The FOAMAT standard software generates both height versus time plots and velocity versus time plots. These plots are useful for comparing the relative reactivity of different catalyst formulations. MDI polyurethane foam was prepared in a conventional hand mix manner. The experiment utilizes 4.8 parts of N,N"-diisopropyl-N,N',N"-trimethyldiethylenetriamine. The shelf life study of the following formulation was tested at 50 °C for four weeks in hot air oven. The rate of rise properties were monitored using FOAMAT sonar. The polyurethane formulations in parts by weight were:

**Table 1: Formulations Used in this example.**

| **Formulation** | **Parts by mass (pphp)** |
|---|---|
| **Polyol^{l,2}** | 100 |
| **Flame retardant³** | 21.5 |
| **Surfactant⁴** | 0.7 |
| **Water** | 2.5 |
| **Catalyst⁵** | 4.8 |
| **HFO⁶** | 10 |
| **HFO solubility enhancer⁷** | 3 |

| | |
|---|---|
| *^{1,2}Polyol : Mixture of polyester (Terol^{®}305) and polyether polyol (Jeffol^{®}R470x) obtained from Huntsman.* *³Flame retardant: TCPP obtained from ICL-IP.* *⁴Surfactant: DABCO*^{®}*DC193 obtained from Evonik Industries.* *⁵Catalyst: N,N"-diisopropyl-N,N',N"-trimethyldiethylenetriamine* *⁶HFO: Solstice^{®} LBA obtained from Honeywell* *⁷HFO solubility enhancer: DABCO*^{®}*PM301 obtained from Evonik Industires.* | |

A homogenous solution of polyol, flame retardant, surfactant, water, catalyst and HFO was placed in a 946 mL (32 ounce) paper cup. MDI was added into it and mixed for 3 seconds at 12,000 RPM using a Laboratory Dispensator made by Premier Mill Corp. Immediately after mixing, the paper cup was placed under the Foamat^{®} machine (FOAMAT Messtechnik GmbH) sonar and software equipment was used to obtain foam rise profiles, and measure the ROR profile for 60 seconds. The three samples of same formulation were kept in three sealed Nalgene bottles and heated at 50 °C for four weeks. The ROR profile was monitored on zero, first, second and fourth week. The summary of the observation is listed in table 2.

**Table 2: Time in Seconds to 80% Maximum Height in Handmix Experiments with HFCO-1233zd(E) Blowing Agent with N,N"-diisopropyl-N,N',N"-trimethyldiethylenetriamine Catalyst.**

| **Week of heat ageing** | **time (Sec)** | **% Change** |
|---|---|---|
| **0** | 16.5 | 0 |
| **1** | 16.5 | 0 |
| **2** | 19.0 | 15.1 |
| **4** | 24.8 | 50.3 |

It is evident from table 2, that the catalyst loses its reactivity profile over the period of 4 weeks at 50°C. However, the catalyst was found to have excellent stability over 2 weeks at 50 °C. It starts losing its reactivity after 2 weeks period at 50°C. At fourth week it lost almost 50% of its original reactivity at 50°C. The chemical reaction of the water and the polyol with the isocyanate is typically catalyzed by both amine and metal catalysts. There are a number of decomposition pathways that can occur in the fully-formulated B-side that can lead to a potential change in system reactivity over time. For example, it has been shown previously that amine catalysts can catalyze the decomposition of HCFC's in polyurethane foam formulations, leading to the production of hydrofluoric or hydrochloric acid which can further decompose the polyester polyols, flame retardants silicone surfactants, the amine or metal catalysts, and lead to loss of efficiency of the blowing agent. Hydrofluoroolefins such as HFCO-1233zd(E) can be additionally challenging due to the electrophilic nature of the sp² carbon of the olefin, making it more susceptible to coordination of the amine catalyst, leading to various possible decomposition pathways.

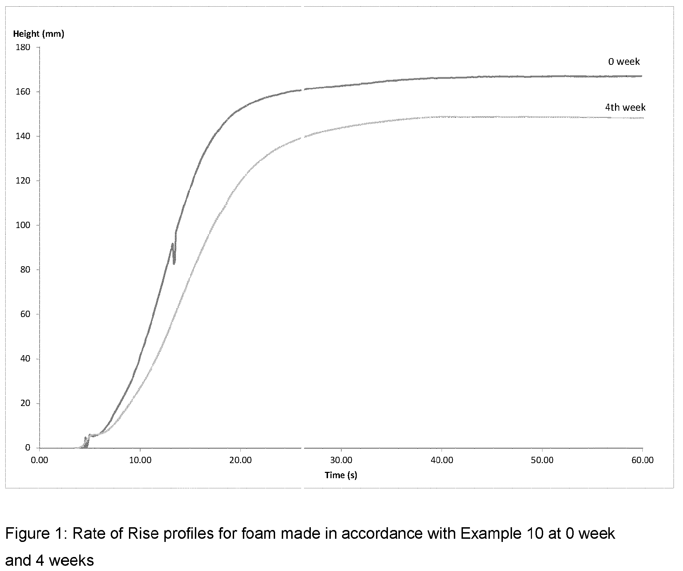

Referring now to Fig. 1, Fig. 1 is a graphical representation in terms of seconds v. mm of the rate of rise for foam made in accordance with Example 10 at 0 week and 4 weeks.

### EXAMPLE 11 (Comparative)

*This example describes the performance of N, N, N', N",N"-pentamethyldiethylenetriamine a standard catalyst used in polyurethane systems for rigid spray foams*

In this example MDI polyurethane foam was prepared in a conventional hand mix in 946 mL (32 ounce) paper cup as described in Example 10. This comparative example utilizes 2.0 pphp of Polycat^{®}-5 a conventional blowing catalyst used in rigid foam applications. The polyester polyol, polyether polyol, flame retardant, water, blowing agent, catalyst, surfactant and HFO solubility enhancer were mixed in a Nalgene container and agitated by shaking by hand until the mixture was well-blended to make a polyol preblend. To make a foam sample for ROR reactivity measurement, 30 g of the polyol pre-blend and 30 g of polymeric MD were combined in a 32 oz paper cup and mixed for 3 seconds at 6000 RPM using an overhead stirrer fitted with a 4 inch diameter stirring blade. The cup was then placed under the FOAMAT sensor. The start time for ROR measurement was automated for the FOAMAT and began directly after end of the mixing. The shelf life study of the following formulation was tested at 50 °C for four weeks in hot air oven. The rate of rise properties were monitored using FOAMAT sonar. The following parts by weight were used in the formulations:

**Table 3. Formulations Used in this example.**

| **Formulation** | **Parts by mass (pphp)** |
|---|---|
| **Polyol^{1,2}** | 100 |
| **Flame retardant³** | 21.5 |
| **Surfactant⁴** | 0.7 |
| **Water** | 0.3 |
| **Catalyst⁵** | 2.0 |
| **HFO⁶** | 10 |
| **HFO solubility enhancer⁷** | 3 |

| | |
|---|---|
| *^{1,2}Polyol : Mixture of polyester (Terol 305) and polyether polyol (R470x) obtained from Huntsman.* *³Flame retardant: TCPP obtained from ICL-IP.* *⁴Surfactant: DABCO*^{®}*DC193 obtained from Evonik Industries.* *⁵Catalyst: Polycat*^{®}*-5 obtained from Evonik Industries.* *⁶HFO: Solstice^{®} LBA obtained from Honeywell* *⁷HFO solubility enhancer: DAABCOOPM301.* | |

Table 3 list the catalyst compositions and table 4 lists the FOAMAT properties obtained after 0 and 4 weeks heat ageing at 50 °C. A homogenous mixture of polyol, flame retardant, surfactant, water, catalyst and HFO was placed in a 946 mL (32 ounce) paper cup. MDI was added into it and mixed for 3 seconds at 6000 RPM. Immediately after mixing, the paper cup was placed under the FOAMAT sonar sensor and measured the ROR profile for 60 seconds.

**Table 4. Time in Seconds to 80% Maximum Height in Handmix Experiments with HFCO-1233zd(E) Blowing Agent with Polycat 5.**

| **Week of heat ageing** | **time (Sec)** | **% Change** |
|---|---|---|
| **0** | 18.6 | 0 |
| **1** | 28.1 | 51 |
| **2** | 36.4 | 95.7 |
| **4** | 56.2 | 202.1 |

It is evident from Table 4 that the formulation loses its activity as shown by a 202% increase in rise time within 4 weeks of heat ageing. This commercially available catalyst is too unstable to be used with HFO blowing agents.

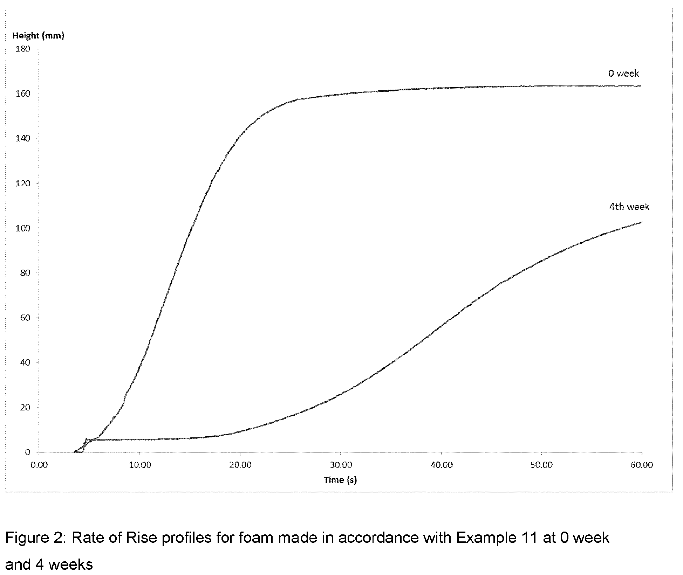

Referring now to Fig. 2, Fig. 2 is a graphical representation in terms of seconds v. mm of the rate of rise for foam made in accordance with Example 11 at 0 week and 4 weeks.

### EXAMPLE 12 (Comparative)

*This example describes the performance of reactive catalyst used in polyurethane systems for rigid spray foams*

In this example, 3.4 parts of the catalyst as defined below were used in order to make the polyurethane. Conventional hand mix foam was made and monitored using FOAMAT free rate of rise software.

**Table 5. Formulations Used in this example.**

| **Formulation** | **Parts by mass (pphp)** |
|---|---|
| **Polyol^{1,2}** | 100 |
| **Flame retardant³** | 21.5 |
| **Surfactant⁴** | 0.7 |
| **Water** | 2.5 |
| **Catalyst⁵** | 3.4 |
| **HFO⁶** | 10 |
| **⁷HFO solubility enhancer** | 3 |

| | |
|---|---|
| *^{1,2}Polyol : Mixture of polyester (Terol^{®}305) and polyether polyol (Jeffol^{®}R470x) obtained from Huntsman.* *³Flame retardant: TCPP obtained from ICL-IP.* *⁴Surfactant: DABCO*^{®}*DC193 obtained from Evonik Industries.* *⁵Catalyst: Mixture of reactive catalysts based on tetramethylguanidine (15%) and N, N, N'-trimethyl-N'-(2-hydroxyethyl)-bis(aminoethyl) ether* (85%) *obtained from Evonik Industries.* *⁶HFO: Solstice*^{®}*LBA obtained from Honewell* *⁷HFO solubility enhancer: DABCO^{®}PM301 obtained from Evonik Industries.* | |

**Table 6: Time in Seconds to 80% Maximum Height in Handmix Experiments with HFCO-1233zd(E) Blowing Agent with a mixture of reactive catalysts.**

| **Week of heat ageing** | **time (Sec)** | **% Change** |
|---|---|---|
| **0** | 18.5 | 0 |
| **1** | 22 | 18.9 |
| **2** | 27.9 | 50.8 |
| **4** | 37.8 | 104.3 |

As shown in table 6, the catalyst loses significant reactivity over the period of four weeks at 50 °C. After four weeks of ageing the rise time increased by 104% from its original value.

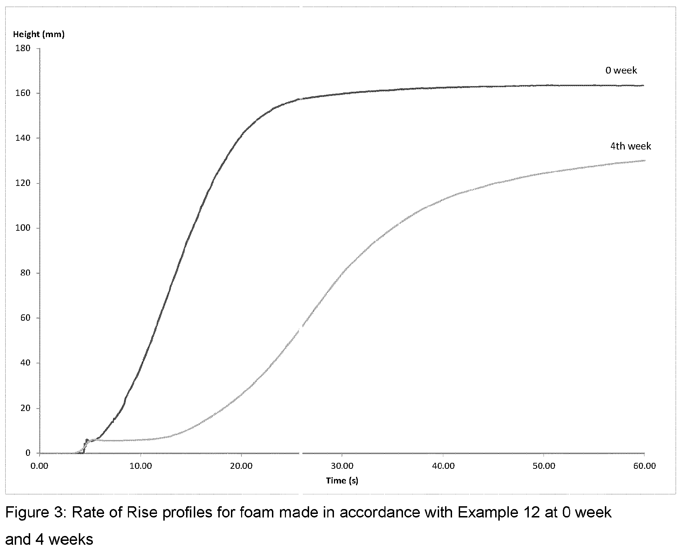

Referring now to Fig. 3, Fig. 3 is a graphical representation in terms of seconds v. mm of the rate of rise for foam made in accordance with Example 12 at 0 week and 4 weeks.

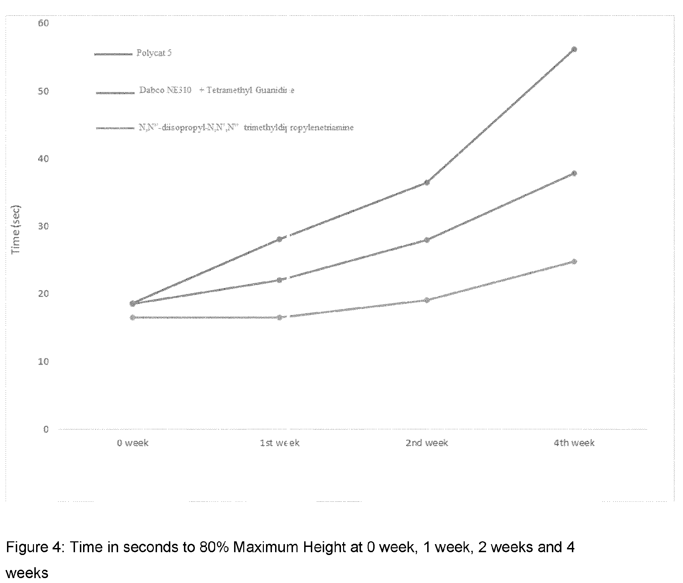

Referring now to Fig. 4, Fig. 4 is a graphical representation of the time in seconds to 80% maximum height at 0 week, 1 week, 2 weeks and 4 weeks for each of (i) Polycat 5, (ii) Dabco NE310 + Tetramethyl Guanidine, and (iii) N,N"-diisopropyl-N,N',N" trimethyldipropylenetriamine.

### EXAMPLE 13 (Comparative)

*This example describes the performance of N, N, N', N",N"-pentamethyldiethylenetriamine in comparison with various catalysts made in previous examples*

In this example MDI polyurethane foam was prepared in a conventional hand mix in a 946 mL (32 ounce) paper cup as described in Example 10. The control experiment utilizes 2.0 parts of pentamethyldiethylenetriamine. The polyester polyol, polyether polyol, flame retardant, water, blowing agent, catalyst, surfactant and HFO solubility enhancer were mixed in a Nalgene container and agitated by shaking by hand until the mixture was well-blended to make a polyol preblend. To make a foam sample for ROR reactivity measurement, 30 g of the polyol pre-blend and 30 g of polymeric MDI were combined in a 32 oz paper cup and mixed for 3 seconds at 6000 RPM using an overhead stirrer fitted with a 4 inch diameter stirring blade. The cup was then placed under the FOAMAT sensor. The start time for ROR measurement was automated for the FOAMAT and began directly after the end of the mixing. The shelf life study of the formulation of example 10 was tested at 50 °C for four weeks in a hot air oven. The rate of rise properties were monitored using FOAMAT sonar. The following parts by weight were used in the formulations for various catalysts:

**Table 7. Catalysts Use Levels For Various Experimental Catalysts**

| **Catalysts Type** | **Name** | **Parts by mass (pphp)** |
|---|---|---|
| Polycat^{®}5 | Pentamethyltriethylenediamine | 2.00 |
| Example-3 Catalyst | N, N', N"-triisopropyldiethylenetriamine | 2.65 |
| Example-2 Catalyst | N, N"-diisopropyldiethylenetriamine | 2.16 |
| Example-5 Catalyst | N, N', N"-triisopropyl-N, N"-dimethyldiethylenetriamine | 2.97 |
| Example-4 Catalyst | N, N"-diisopropyl-N, N', N"-trimethyldiethylenetriamine | 2.65 |
| Example-6 Catalyst | N, N"-bis(2-hydroxypropyl)-N, N', N"-triisopropyldiethylenetriamine | 3.99 |
| Example-7 Catalyst | N, N', N"-tri(2-hydroxypropyl)-N, N"-diisopropyldiethylenetriamine | 4.17 |

Table 7 lists the catalyst compositions and and the use levels employed for a 4 weeks heat ageing study at 50 °C. In this example, Polycat^{®}-5 was chosen to represent a standard blowing catalyst for hydrofluoro olefin (HFO) based formulations. A homogenous mixture of polyol, flame retardant, surfactant, water, catalyst and HFO was placed in a 946 mL (32 ounce) paper cup. MDI was added into it and mixed for 3 seconds at 6000 RPM. Immediately after mixing, the paper cup was placed under the FOAMAT sonar sensor and measured the ROR profile for 60 seconds.

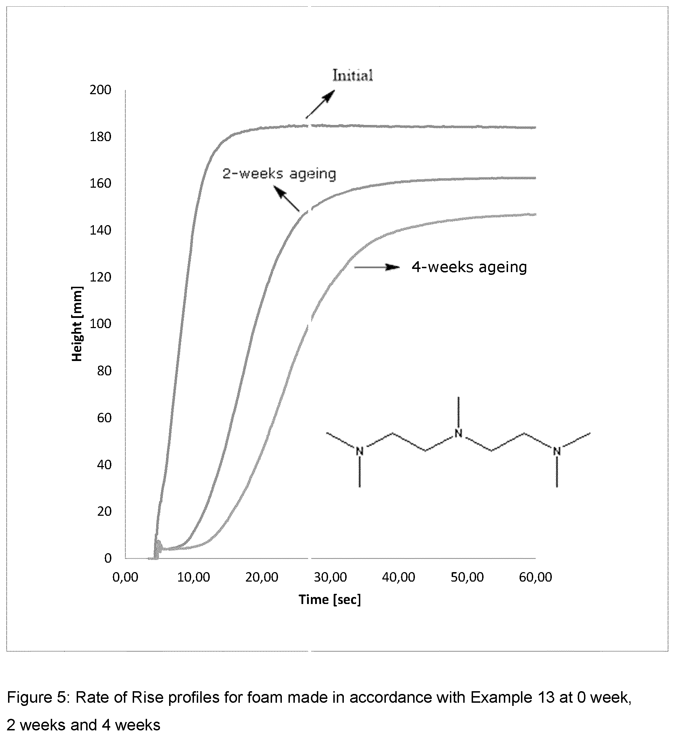

Referring now to Fig. 5, Fig. 5 is a graphical representation in terms of the foam height (mm) *vs.* time (seconds) for a foam made with Polycat^{®}5 (pentamethyltriethylenediamine) at the initial stage as well as the rise profile resulting from 2 weeks and 4 weeks thermal ageing. Substantial loss in foam height and rise speed is observed after ageing for 2 and 4 weeks due to catalyst deactivation.

After 4 weeks of aging the system based on Polycat^{®}5 had the cell structure deteriorated substantially resulting in a large loss in foam height. Also a reactivity drift was observed with aging resulting in 13 seconds delay after 2 weeks of ageing and 21 seconds delay after 4 weeks of ageing.

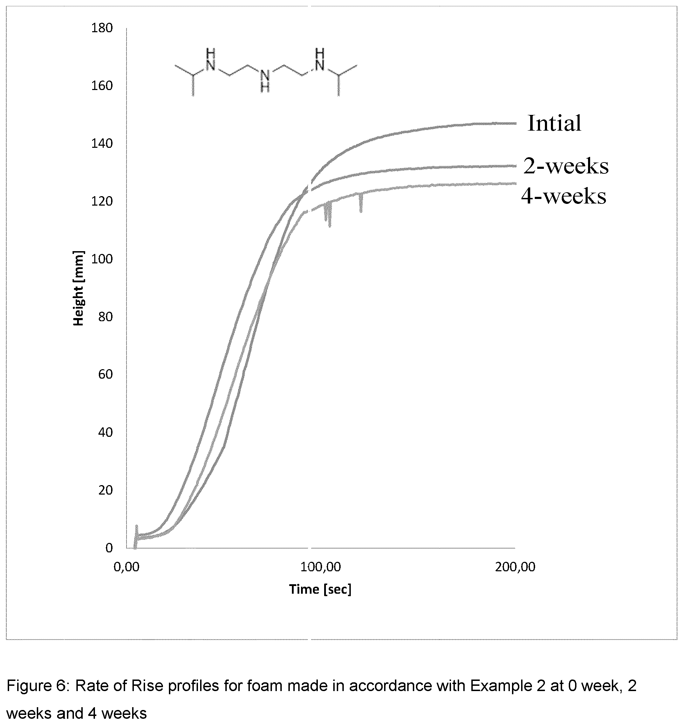

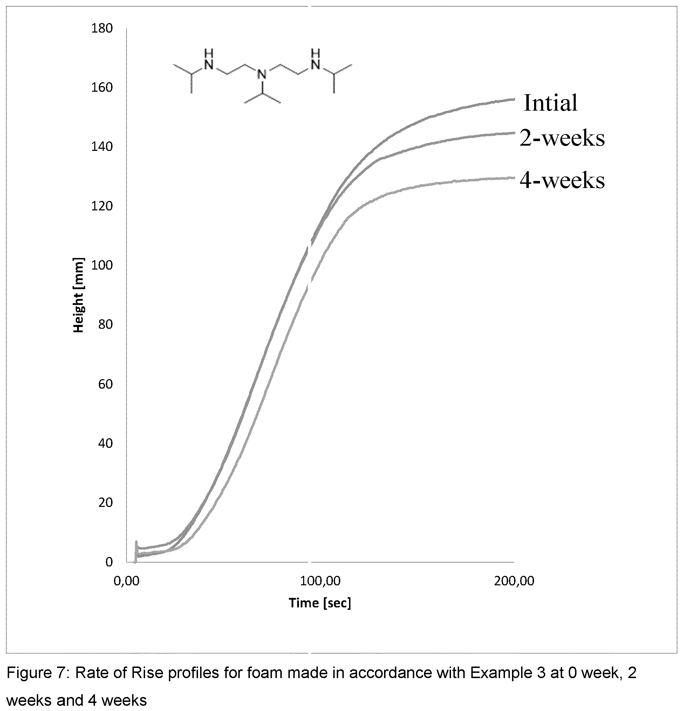

Referring now to Figs. 6 and 7, Figs. 6 and 7 are graphical representations in terms of the foam height (mm) *vs.* time (seconds) for a foam made with catalysts of Example 2 and Example 3 respectively, at the initial stage as well as the rise profile resulting from 2 weeks and 4 weeks thermal ageing. In this case due to the presence of reactive sites on each molecule, the catalytic activity of these amines is decreased substantialy in comparison to Polycat^{®} 5 showing about 100 seconds delay for the catalyst of Example 3 and about 75 seconds delay for the catalysts of Example 2. The reactivity drift over ageing is minimal probably due to the fact that these amines react rapidly with HFOs leading to possible surfactant degradation. A sign of their poor catalytic acitivity is illustrated by their significant decrease in foam height and poor and coarse cell structure.

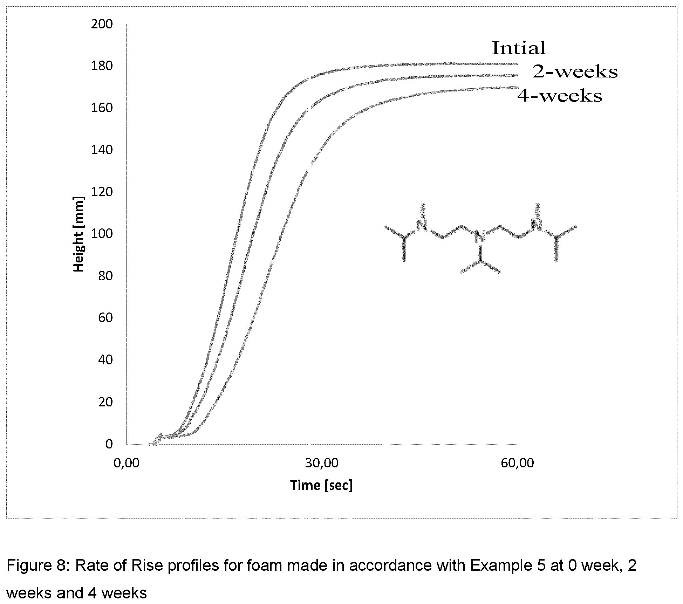

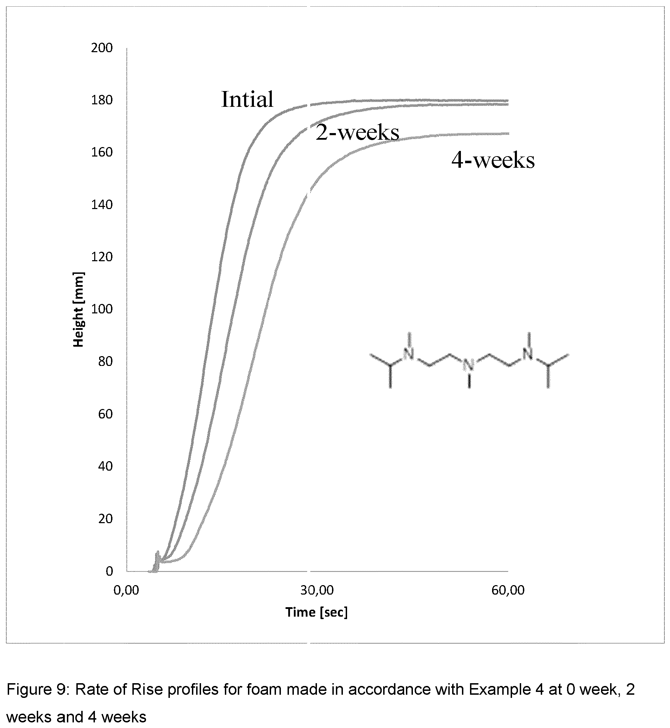

Referring now to Figs. 8 and 9, Figs. 8 and 9 are graphical representations illustrating the impact on HFO-based system stability for catalysts of example 5 and example 4 respectively. The catalytic efficiency of N, N', N"-triisopropyl-N, N"-dimethyldiethylenetriamine (example 5) is substantially lower (4.17 pphp) due to the presence of an isopropyl group at the central nitrogen of the diethylenetriamine backbone than the catalytic efficiency of N, N"-diisopropyl-N, N', N"-dimethyldiethylenetriamine (example 4) where the use level is 3.72 pphp. Thus, substituting the central nitrogen of the diethylenetriamine backbone with an isopropyl group instead of a methyl group results in a use level increase from 3.72 pphp to 4.17 pphp. This large increase in use level nevertheless results in a minor improvement in system stability as can be seen in Figs. 8 and 9.

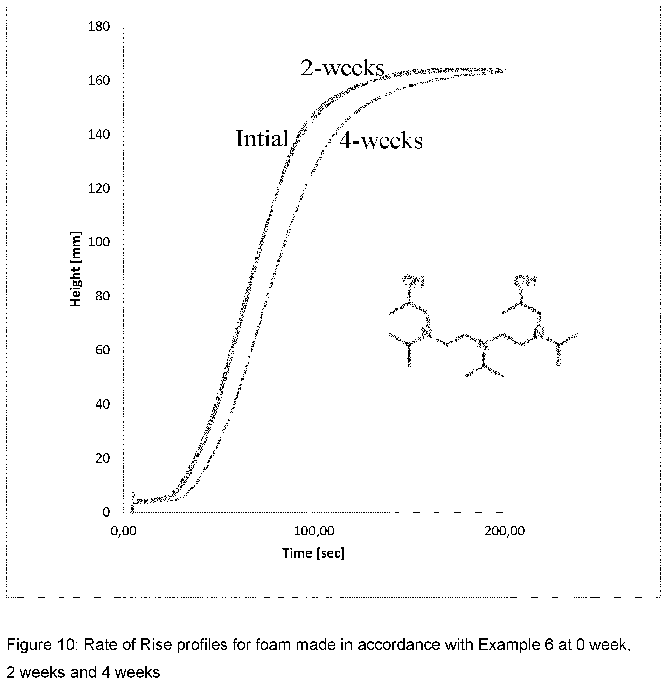

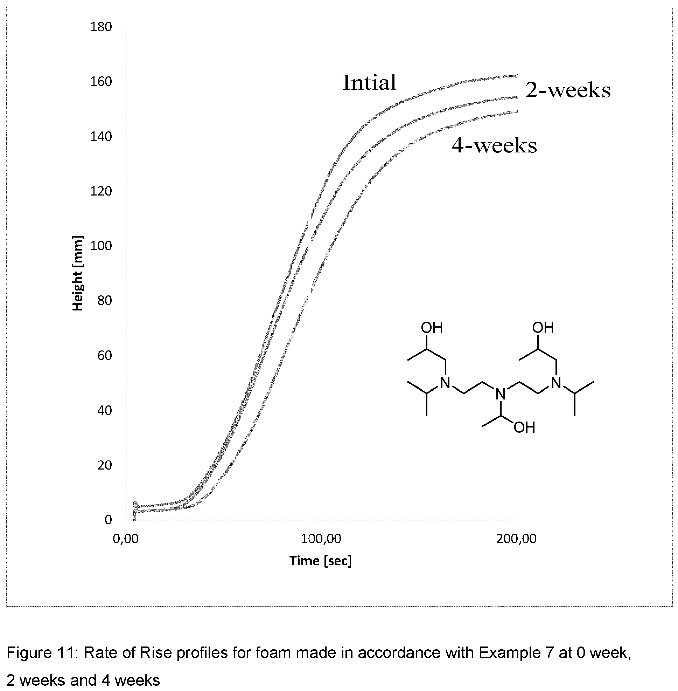

For the case of catalysts of examples 6 and 7, due to the isocyanate reactive OH-group on each molecule and the steric hindrance around each nitrogen atom, the catalytic activity is decreased significantly in comparison to standard Polycat^{®}-5 causing about 100 seconds delay for N, N"-bis(2-hydroxypropyl)-N, N', N"-triisopropyldiethylenediamine even at a use level of 5.60 pphp (2x use level of Polycat^{®}-5) and about 75 seconds delay for N, N', N"-tri(2-hydroxypropyl)-N, N"-diisopropyldiethylenediamine even at a use level of 5.86 pphp (2.1x use level of Polycat^{®}-5). During ageing of premixes having these catalysts, the foam rise kinetic data showed a one second drift after 2 weeks of aging and further HFO-system degradation occurred after 4 weeks of aging. Since further use level increases are needed to meet the foam kinetics of the standard these candidates are expected to substantially alter the composition of the foam formulation resulting in poor quality foam products. Because no substantial reaction speed can be achieved at reasonable amine-catalyst use levels these compounds were not suitable for practical use. Referring now to Figs. 10 and 11, Figs. 10 and 11 are graphical representations illustrating the impact on HFO-based system stability for catalysts of example 6 and example 7 respectively.

### EXAMPLE 14 (Non-Inventive)

*This example describes the performance of N, N, N', N",N"-pentamethyldiethylenetriamine in comparison with various catalysts made in previous examples*

The shelf life study was carried out in the same maner as in example 13 using various catalysts:

**Table 8. Catalysts Use Levels For Various Experimental Catalysts**

| **Catalysts Type** | **Name** | **Wt. %** |
|---|---|---|
| Polycat^{®}5 | Pentamethyltriethylenediamine | 2.00 |
| Example-5 Catalyst | N, N', N"-triisopropyl-N, N"-dimethyldiethylenetriamine | 4.40 |
| Example-4 Catalyst | N, N"-diisopropyl-N, N', N"-trimethyldiethylenetriamine | 3.50 |
| Example-1 Catalyst | N-isopropyl-N, N', N", N"-tetramethyldiethylenetriamine | 3.25 |
| Example-8 Catalyst | N,N"-diisopropyl-N,N',N"-triethyldiethylenetriamine | 10.73 |

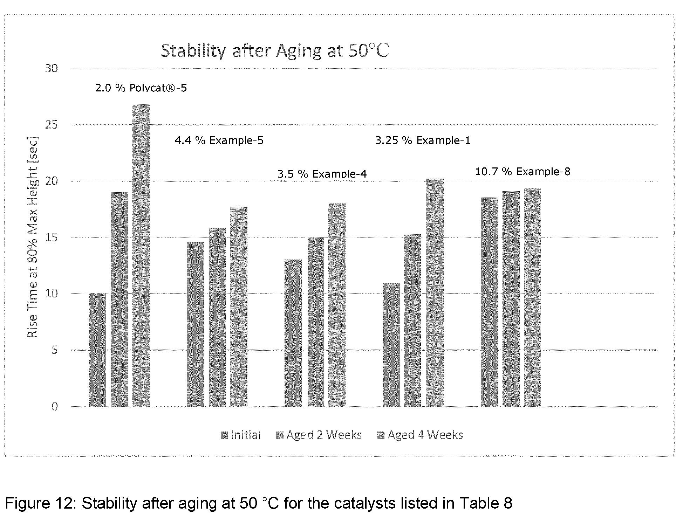

Table 8 lists the catalyst names and example references as well as the use levels employed for a 4 weeks heat ageing comparative study at 50 °C. Polycat 5 was used as representative blowing catalyst for hydrofluoro olefin (HFO) based formulations. Foam samples were prepared in mxing cups as described in the previous examples. Referring now to Fig. 12, Fig. 12 is a bar diagram representing the stability after aging at 50 °C for the catalysts listed in Table 8. The height of the bar diagrams represent the rise time height measured at 80 % total foam height in seconds. Each set of bars represents the measurement for a single catalyst where the left bar represents the initial rise time, the middle bar represents the rise time after two weeks of ageing and the right bar represents the rise height after four weeks of ageing. Use levels for each catalyst are expressed in wt.% in the formulation and it is shown above each bar set. From the data it can be seen that Polycat^{®}-5 rise time increases substantially with ageing indicating slowing down of the foaming process. The diagram also shows that as the steric hindrance of the catalyst increases so does the use level. For example, the catalyst of example 8 where substituents at the nitrogen atom are C₂-hydrocarbon (ethyl) and C₃-hydrocarbon (isopropyl) requires very high use levels. Even when the use level is higher than 10 % of the formulation the catalyst is still unable to provide a rise time comparable to Polycat^{®}-5. This means that this composition has been essentially deactivated and therefore no substantial HFO deterioration occurs. At the same time, the foam is not able to provide useful kinetics. The catalysts of example-1, example-4 and example-5 show that as the number of isopropyl groups increases (from 1 to 2 to 3 respectively) the use level increases (from 3.25 wt.% to 3.5 wt.% to 4.4 wt.%). Although the catalyst of example-1 can provide acceptable kinetics, its deterioration over time is quite substantial producing foam of poor quality after ageing. The catalyst of example 5 provides good ageing stability but the use level is still high. The best candidate identified in this example is the catalyst of example-4 where the use level is below 4.0 wt.% but its ageing stability is quite comparable to the stability provided by the catalyst of example-5.

### EXAMPLE 15 (Comparative)

*This example shows that excessive steric hindrance in N-methyl-N,N-dicyclohexylamine results in very high use levels to achieve foam rise kinetics comparable to standards*

The use level was measured in a conventional rigid foam formulation using a physical blowing agent HFC-245fa

**Table 9. Rigid Foam Formulation**

| **Comonent** | **Formulat 1 Wt. %** | **Formula 2 Wt. %** |
|---|---|---|
| Polyol*¹* | 87.5 | 87.5 |
| Polycat^{®}-8*²* | 1.50 | ----- |
| Polycat^{®}-12*³* | ----- | 8.00 |
| HFC-245fa*⁴* | 11.30 | 11.30 |
| Silicone Surfactant*⁵* | 2.00 | 2.00 |
| Rubinate^{®}M*⁶*: Resine ratio | 100:92 | 100:92 |

| | | |
|---|---|---|
| *¹Polyol: Conventional Polyol* *²Polycat*^{®}*-8: commercial catalyst supplied by Evonik Corporation; N-Cyclohexyl-N,N-Dimethylamine* *³Polycat*^{®}*-12: N,N-Dicyclohexyl-N-Methylamine* *⁴Enovate*^{®}*HFC-245fa: a physical blowing agent commercially available from Honeywell; 1,1,1,3,3-pentafluoropropane* *⁵Silicone Surfactant* *⁶Ruminate*^{®}*M: polymeric MDI (methylene diphenyl diisocyanate) supplied by Huntsman with 31.2 % NCO content* | | |

Table 10 below illustrates that when Polycat^{®}-8 (N-cyclohexyl-N,N-diethylamine) is used in a standard formulation a relatively small amount of catalysts (1.5 pphp) is sufficient to ensure adequate kinetics for foam processing. However, in the case of Polycat^{®}-12 when a methyl-group of Polycat^{®}-8 is replaced with another cyclohexyl group the use level increases to 8.00 pphp to achieve a similar string gel time or the equivalent of a more than 5x the use level of Polycat^{®}-8.

**Table 10. Catalyst Use Levels for Polycat^{®}-8 and Polycat^{®}-12**

| **Formula** | **Catalyst** | **Catalyst pphp** | **Cream Time¹ (seconds)** | **String Gel Time (seconds)** |
|---|---|---|---|---|
| 1 | Polycat-8 | 1.50 | 12 | 76 |
| 1 | Polycat-8 | 1.50 | 12 | 74 |
| 2 | Polycat-12 | 8.00 | 11 | 76 |
| 2 | Polycat-12 | 8.00 | 11 | 74 |

| | | | | |
|---|---|---|---|---|
| *¹Cream time: the time in seconds indicating the beginning of the reaction identified by a change in the liquid appearance* | | | | |

### EXAMPLE 16 (Comparative)

*This example shows the improvement in HFO shelf life when catalyst bis(2-pyrrolidinoethyl)ether (BDPE) is used in a premix formulation (an alternative names for bis(2-pyrrolidinoethyl)ether is 1, 1'-(oxybis(ethane-2-1-diyl) dipyrrolidine)*

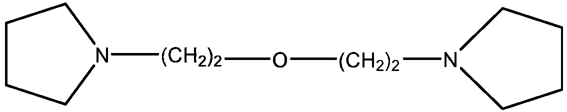

In this example, 2.5 parts of BDPE was used as a blowing-catalyst in order to make the polyurethane. Conventional hand mix foam was made and monitored using FOAMAT free rate of rise software.

**Table 11. Formulations Used in this example.**

| **Formulation** | **Parts by mass (pphp)** |
|---|---|
| **Polyol^{l,2}** | 100 |
| **Flame retardant³** | 21.5 |
| **Surfactant⁴** | 0.7 |
| **Water** | 2.5 |
| **Catalyst⁵** | 2.5 |
| **HFO⁶** | 10 |
| **HFO solubility enhancer⁷** | 3 |

| | |
|---|---|
| *^{1,2}Polyol : Mixture of polyester (Terol^{®}305) and polyether polyol (Jeffol*^{®}*R470x) obtained from Huntsman.* *³Flame retardant: TCPP obtained from ICL-IP.* *⁴Surfactant: DABCO*^{®}*DC193 obtained from Evonik Industries.* *⁵Catalyst: 1,1'-(oxybis(ethane-2-1-diyl) dipyrrolidine* *⁶HFO: Solstice*^{®}*LBA obtained from Honewell* *⁷HFO solubility enhancer: DABCO*^{®}*PM301 obtained from Evonik Industries.* | |

**Table 12: Time in Seconds to 80% Maximum Height in Handmix Experiments with HFCO-1233zd(E) Blowing Agent with BDPE after 5 days at 60°C.**

| **Number of days** | **time (Sec)** | **% Change** |
|---|---|---|
| **0** | 15 | 0 |
| **5** | 19.3 | 28.6 |

It is evident from table 12, that the catalyst *BDPE* provides stability towards HFOs. At the 5^{th} day at 60C it lost ~28.6 % of its original reactivity.

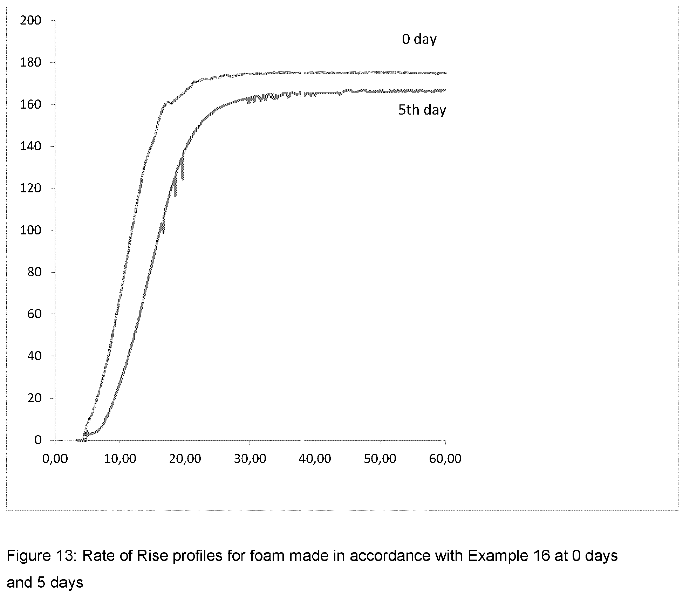

Referring now to Fig. 13, Fig. 13 is a graphical representation in terms of seconds v. mm of the rate of rise for foam made in accordance with Example 16 at 0 days and 5 days.

### EXAMPLE 17 (Comparative)

*This example shows the improvement in HFO shelf life when catalyst N,N"-diisopropyl-N,N',N"-trimethyldiethylenetriamine is used in combination with 2,2'-dimorpholinodiethyl ether*

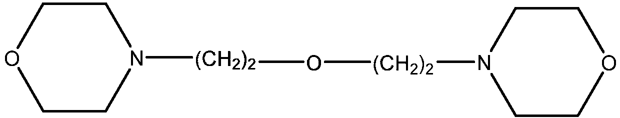

In this example, a combination of 4.8 parts of N,N"-diisopropyl-N,N,N"-trimethyldiethylenetriamine and 2.0 parts of Dabco^{®}DMDEE was used as catalyst. Conventional hand mix foam was made and monitored using FOAMAT free rate of rise software.

**Table 13. Formulations Used in this example.**

| **Formulation** | **Parts by mass (pphp)** |
|---|---|
| **Polyol^{1,2}** | 100 |
| **Flame retardant³** | 21.5 |
| **Surfactant⁴** | 0.7 |
| **Water** | 2.5 |
| **Catalyst⁵** | 4.8 |
| **Catalyst ⁶** | 2.0 |
| **HFO⁷** | 10 |
| **HFO solubility enhancer⁸** | 3 |

| | |
|---|---|
| *^{1,2}Polyol : Mixture of polyester (Terol*^{®}*305) and polyether polyol (Jeffol^{®}R470x) obtained from Huntsman.* *³Flame retardant: TCPP obtained from ICL-IP.* *⁴Surfactant: DABCO*^{®}*DC193 obtained from Evonik Industries.* *⁵Catalyst: N,N"-diisopropyl-N,N',N" trimethyldiethylenetriamine.* *⁶Catalyst: Dabco*^{®}*DMDEE obtained from Evonik Industries (2,2'-Dimorpholinodiethyl ether).* *⁷HFO: Solstice*^{®}*LBA obtained from Honeywell* *⁸HFO solubility enhancer: DABCO*^{®}*PM301 obtained from Evonik Industries..* | |

**Table 14: Time in Seconds to 80% Maximum Height in Handmix Experiments with HFCO-1233zd(E) Blowing Agent and Combination of N,N"-diisopropyl-N,N',N" trimethyldiethylenetriamine and DABCO^{®}DMDEE.**

| **Week of heat ageing** | **time (Sec)** | **% Change** |
|---|---|---|
| **0** | 15.2 | 0 |
| **1** | 13.4 | -11.0 |
| **2** | 15.4 | 1.32 |
| **4** | 20.0 | 31.5 |

As shown in Table 14 catalyst N,N"-diisopropyl-N,N',N" trimethyldiethylenetriamine when used in combination with Dabco^{®}DMDEE provides additional stability to hydrofluoroolefins. At fourth week it lost only 31% of its original reactivity at 50 °C.

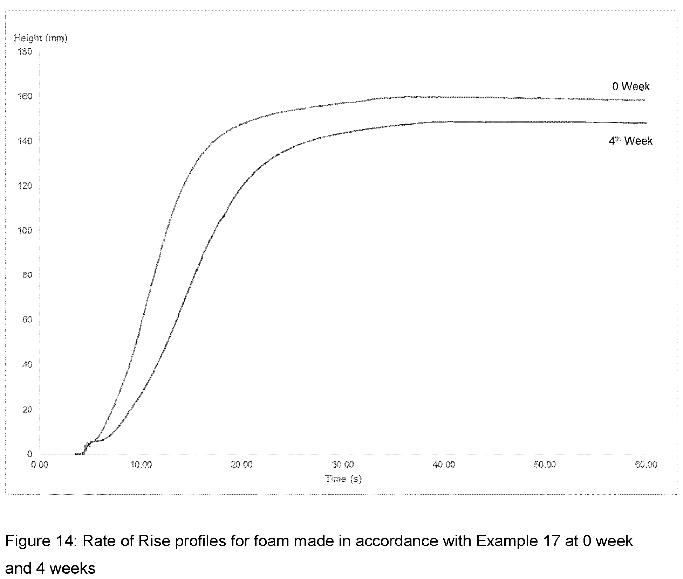

Referring now to Fig. 14, Fig. 14 is a graphical representation in terms of seconds v. mm of the rate of rise for foam made in accordance with Example 17 at 0 week and 4 weeks.

### EXAMPLE 18 (Non-Inventive)

*This example shows the improvement in HFO shelf life when catalysts N,N"-diisopropyl-N,N',N"-trimethyldiethylenetriamine is used in combination with 1,2-dimethylimidazole*

In this example, a combination of 2.0 parts of N,N"-diisopropyl-N,N',N" trimethyldipropylenetriamine and 2.0 parts of DABCO^{®}2040 (1,2-dimethyl imidazole in diethylene glycol) was used to improve the shelf life of HFO-system premix. Conventional hand mix foam was made and monitored using FOAMAT free rate of rise software.

**Table 15. Formulations Used in this example.**

| **Formulation** | **Parts by mass (pphp)** |
|---|---|
| **Polyol^{1,2}** | 100 |
| **Flame retardant³** | 21.5 |
| **Surfactant⁴** | 0.7 |
| **Water** | 2.5 |
| **Catalyst⁵** | 2.0 |
| **Catalyst ⁶** | 2.0 |
| **HFO⁷** | 10 |
| **HFO solubility enhancer⁸** | 3 |

| | |
|---|---|
| *^{1,2}Polyol : Mixture of polyester (Terol*^{®}*305) and polyether polyol (Jeffol^{®}R470x) obtained from Huntsman.* *³Flame retardant: TCPP obtained from ICL-IP.* *⁴Surfactant: DABCO*^{®}*DC193 obtained from Evonik Industries.* *⁵Catalyst: N,N"-diisopropyl-N,N',N" trimethyldiethylenetriamine.* *⁶Catalyst: Dabco*^{®}*2040 obtained from Evonik Industries (1,2-dimethylimidazole).* *⁷HFO: Solstice*^{®}*LBA obtained from Honeywell* *⁸HFO solubility enhancer: DABCO*^{®}*PM301 obtained from Evonik Industries..* | |

**Table 16: Time in Seconds to 80% Maximum Height in Handmix Experiments with HFCO-1233zd(E) Blowing Agent and Combination of N,N"-diisopropyl-N,N',N" trimethyldiethylenetriamine and 1,2-Dimethylimidazole.**

| **Week of heat ageing** | **time (Sec)** | **% Change** |
|---|---|---|
| **0** | 19.3 | 0 |
| **1** | 18.4 | -4.6 |
| **2** | 22 | 14 |
| **4** | 28.3 | 46.6 |

As shown in Table 16, N,N"-diisopropyl-N,N',N" trimethyldiethylenetriamine used in combination with DABCO^{®}2040 (dimethyl imidazole in diethylene glycol) provided additional stability of hydrochlorofluoroolefins premix systems. After ageing for 4 weeks at 50 °C only a 46 % increase in the time to reach 80% of the maximum height was observed.

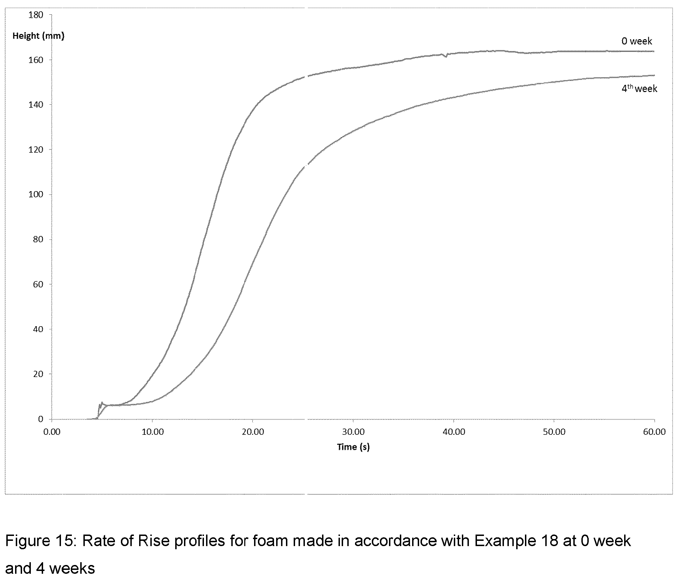

Referring now to Fig. 15, Fig. 15 is a graphical representation in terms of seconds v. mm of the rate of rise for foam made in accordance with Example 18 at 0 week and 4 weeks.

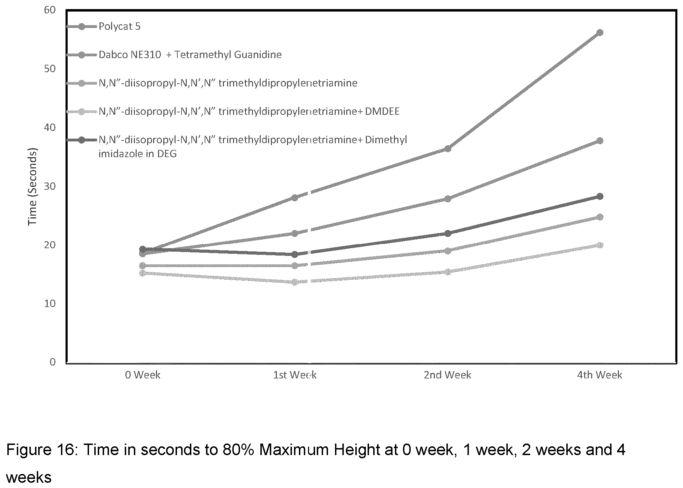

Referring now to Fig. 16, Fig. 16 is a graphical representation of the time in seconds to 80% maximum height at 0 week, 1 week, 2 weeks and 4 weeks for each of (i) Polycat 5, (ii) a mixture of Dabco NE310 and Tetramethyl Guanidine, (iii) N,N"-diisopropyl-N,N',N" trimethyldipropylenetriamine, (iv) a mixture of N,N"-diisopropyl-N,N',N" trimethyldipropylenetriamine and DMDEE, and (v) a mixture of N,N"-diisopropyl-N,N',N" trimethyldipropylenetriamine and Dimethyl imidazole in DEG.

### EXAMPLE 19 (Comparative)

In this example, 2.5 parts of BDPE was used as a blow-catalyst in order to make the polyurethane. Conventional hand mix foam was made and monitored using FOMAT free rate of rise software.

**Table 17. Formulations Used in this example.**

| **Formulation** | **Parts by mass (pphp)** |
|---|---|
| **Polyol^{l,2}** | 100 |
| **Flame retardant³** | 21.5 |
| **Surfactant⁴** | 0.7 |
| **Water** | 2.5 |
| **Catalyst⁵** | 2.5 |
| **HFO⁶** | 10 |
| **HFO solubility enhancer⁷** | 3 |

| | |
|---|---|
| *^{1,2}Polyol : Mixture of polyester (Terol*^{®}*305) and polyether polyol (R470x) obtained from Huntsman.* *³Flame retardant: TCPP obtained from ICL-IP.* *⁴Surfactant: DC193 obtained from Evonik Industries.* *⁵Catalyst: BDPE .* *⁶HFO: Solstice LBA* *⁷HFO solubility enhancer: PM301.* | |

**Table 18: Time in Seconds to 80% Maximum Height in Handmix Experiments with HFCO-1233zd(E) Blowing Agent with BDPE after 4 weeks at 50 °C.**

| **Week of heat ageing** | **time (Sec)** | **% Change** |
|---|---|---|
| **0** | 15 | 0 |
| **1** | 16 | 6.6 |
| **2** | 22 | 46.6 |
| **4** | 28 | 86.6 |

It is evident from table 18, that the catalyst BDPE provides moderate stability towards HFOs. After 4 weeks at 50°C it lost ~86.6 % of its original reactivity.

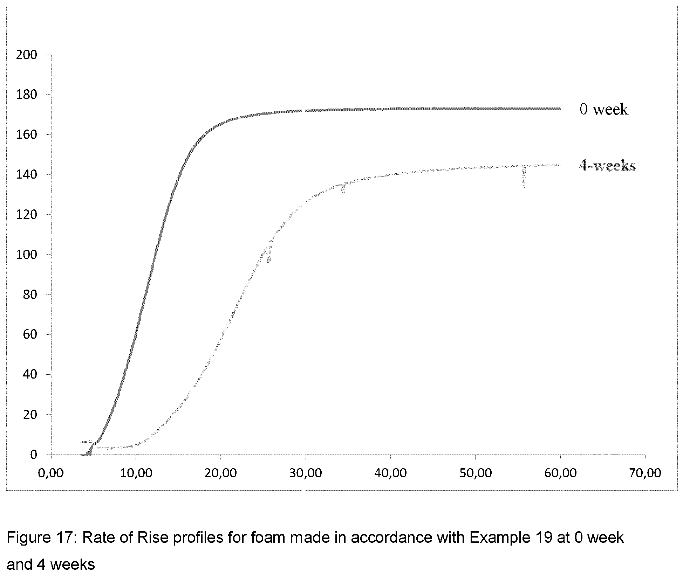

Referring now to Fig. 17, Fig. 17 is a graphical representation in terms of seconds v. mm of the rate of rise for foam made in accordance with Example 19 at 0 week and 4 weeks.

### EXAMPLE 20

In this example, 2.5 parts of BDPE and 1.0 parts of DMDEE was used as a blow-catalyst in order to make the polyurethane. Conventional hand mix foam was made and monitored using FOMAT free rate of rise software.

**Table 19. Formulations Used in this example.**

| **Formulation** | **Parts by mass (pphp)** |
|---|---|
| **Polyol^{1,2}** | 100 |
| **Flame retardant³** | 21.5 |
| **Surfactant⁴** | 0.7 |
| **Water** | 2.5 |
| **Catalyst⁵** | 2.5 |
| **Catalyst⁶** | 1.0 |
| **HFO⁷** | 10 |
| **HFO solubility enhancer⁸** | 3 |

| | |
|---|---|
| *^{1,2}Polyol : Mixture of polyester (Terol 305) and polyether polyol (R470x) obtained from Huntsman.* *³Flame retardant: TCPP obtained from ICL-IP.* *⁴Surfactant: DC193 obtained from Evonik Industries.* *⁵Catalyst: BDPE .* *⁶Catalyst: DABCO DMDEE* *⁷HFO: Solstice LBA* *⁸HFO solubility enhancer: PM301.* | |

**Table 20: Time in Seconds to 80% Maximum Height in Handmix Experiments with HFCO-1233zd(E) Blowing Agent with BDPE after 4 weeks days at 50°C.**

| **Week of heat ageing** | **time (Sec)** | **% Change** |
|---|---|---|
| **0** | 15 | 0 |
| **1** | 18 | 20 |
| **2** | 21 | 40 |
| **4** | 29 | 93.3 |

It is evident from table 20, that the catalysts combination of BDPE and DMDEE does not provides any additional stability towards HFOs. After 4 weeks at 50C it lost ~93.3 % of its original reactivity.

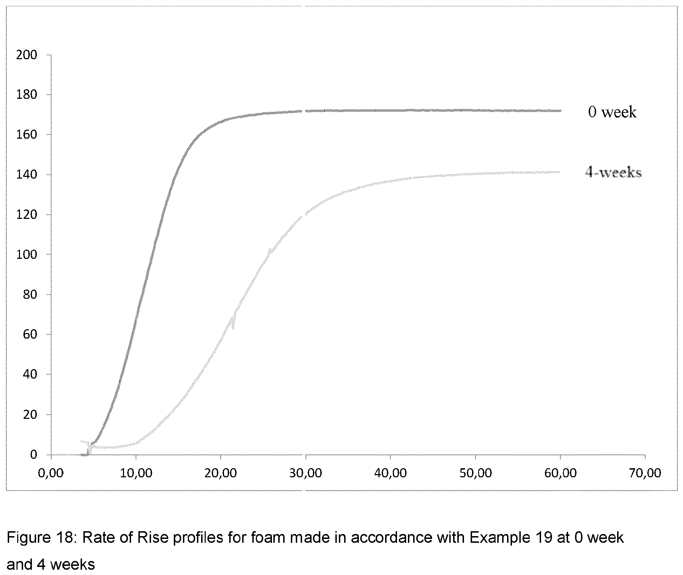

Referring now to Fig. 18, Fig. 18 is a graphical representation in terms of seconds v. mm of the rate of rise for foam made in accordance with Example 20 at 0 week and 4 weeks.

### EXAMPLE 21 (Non-Inventive)

Emission profile of N,N"-diisopropyl-N,N',N"-trimethyldipropylenetriamine and BDPE ASTM D8142-17 Microchamber Emission test was performed on handmix spray polyurethane foam samples. Details of the micro-chamber parameters are listed in Table 21. After completion of sampling, Tenax TD tubes were further desorbed and contents analyzed using thermal desorption GC/MS. It is evident from the test that N,N"-diisopropyl-N,N',N"-trimethyldipropylenetriamine and BDPE are non-emissive amine catalysts.

**Table 21. Catalyst Emission Results**

| Area Specific Emission Rates (µg/m²hr) | | |
|---|---|---|
| | N,N"-diisopropyl-N,N',N"-trimethyldipropylenetriamine | BDPE |

| 2 hrs. | | |
|---|---|---|
| Prep 1 | ND | ND |
| Prep 2 | ND | ND |

| 24 hrs. | | |
|---|---|---|
| Prep 1 | ND | ND |
| Prep 2 | ND | ND |

| 48 hrs. | | |
|---|---|---|
| Prep 1 | ND | ND |
| Prep 2 | ND | ND |

**Table 22. Estimated detection limits**

| Compoud | Method Detection limit on TD tube, ng |
|---|---|
| N,N"-diisopropyl-N,N',N"-trimethyldipropylenetriamine | 15.3 |
| BDPE | 10.7 |

## Claims

1. A catalyst composition comprising at least one compound selected from the group consisting of N, N'-dipropyl-N, N'-dimethyl-bis(aminoethyl)ether; N, N'-diisopropyl-N, N'-dimethyl-bis(aminoethyl)ether; N, N'-dibutyl-N, N'-dimethyl-bis(aminoethyl)ether; N, N'-diisobutyl-N, N'-dimethyl-bis(aminoethyl)ether; N, N'-disecbutyl-N, N'-dimethyl-bis(aminoethyl)ether; N, N'-ditertbutyl-N, N'-dimethyl-bis(aminoethyl)ether; N, N'-dipentyl-N, N'-dimethyl-bis(aminoethyl)ether; N, N'-ditertpentyl-N, N'-dimethyl-bis(aminoethyl)ether; N, N'-dineopentyl-N, N'-dimethyl-bis(aminoethyl)ether; N, N'-disecpentyl-N, N'-dimethyl-bis(aminoethyl)ether; N, N'-di(3-pentyl)-N, N'-dimethyl-bis(aminoethyl)ether; N, N'-disecisoopentyl -N, N'-dimethyl-bis(aminoethyl)ether; N, N'-dihexyl-N, N'-dimethyl-bis(aminoethyl)ether; N, N'-diisohexyl-N, N'-dimethyl-bis(aminoethyl)ether; and N, N'-dineohexyl-N, N'-dimethyl-bis(aminoethyl)ether.

2. The catalyst composition of claim 1 further comprising a metal catalyst, a tertiary amine catalyst having or not an isocyanate reactive group, or a combination thereof.

3. The catalyst composition of claim 2, wherein the tertiary amine has at least one isocyanate reactive group comprising a primary hydroxyl group, a secondary hydroxyl group, a primary amine group, a secondary amine group, a urea group or an amide group.

4. The catalyst composition of claim 3, wherein the tertiary amine is selected from the group consisting of N, N-bis(3-dimethylaminopropyl)-N-isopropanolamine; N, N-dimethylaminoethyl-N'-methyl ethanolamine; N, N, N'-trimethylaminopropylethanolamine; N, N-dimethylethanolamine; N, N-diethylethanolamine; N, N-dimethyl-N', N'-2-hydroxy(propyl)-1,3-propylenediamine; dimethylaminopropylamine; (N, N-dimethylaminoethoxy) ethanol; methyl-hydroxy-ethyl-piperazine; bis(N, N-dimethyl-3-aminopropyl) amine; N, N-dimethylaminopropyl urea; diethylaminopropyl urea; N, N'-bis(3-dimethylaminopropyl)urea; N, N'-bis(3-diethylaminopropyl)urea; bis(dimethylamino)-2-propanol; 6-dimethylamio-1-hexanol; N-(3-aminopropyl) imidazole); N-(2-hydroxypropyl) imidazole; N-(2-hydroxyethyl) imidazole; 2-[N-(dimethylaminoethoxyethyl)-N-methylamino] ethanol; N, N-dimethylaminoethyl-N'-methyl-N'-ethanol; dimethylaminoethoxyethanol; N, N, N'-trimethyl-N'-3-aminopropyl-bis(aminoethyl) ether; or a combination thereof.

5. The catalyst composition of claim 1 wherein the catalyst composition is acid blocked with a carboxylic or sulfonic acid.

6. The catalyst composition of claim 5 wherein the composition is acid blocked with an acid selected from the group consisting of formic acid, acetic acid, propionic acid, butanoic acid, pentanoic acid, neopentanoic acid, hexanoic acid, 2-ethylhexyl carboxylic acid, neohexanoic acid, octanoic acid, neooctanoic acid, heptanoic acid, neoheptanoic acid, nonanoic acid, neononanoic acid, decanoic acid, neodecanoic acid, undecanoic acid, neoundecanoic acid, dodecanoic acid, neododecanoic acid, myristic acid, pentadecanoic acid, hexadecanoic acid, heptadecanoic acid, octadecanoic acid, benzoic acid, oxalic acid, malonic acid, succinic acid, glutaric acid, adipic acid, pimelic acid, suberic acid, azelaic acid, sebacic acid, glycolic acid, lactic acid, tartaric acid, citric acid, malic acid, and salicylic acid.

7. The catalyst composition of claim 1 further comprising catalytic materials, wherein the catalytic materials are selected from the group consisting of potassium formate, potassium acetate, potassium propionate, potassium butanoate, potassium pentanoate, potassium hexanoate, potassium heptanoate, potassium octoate, potassium 2-ethylhexanoate, potassium decanoate, potassium butyrate, potassium isobutyrate, potassium nonante, potassium stearate, sodium octoate, lithium stearate, sodium caprioate, lithium octoate, 2-hydroxypropyltrimethylammonium octoate solution, or any combination thereof.

8. A premix comprising the contact product of at least one active hydrogen-containing compound, at least one blowing agent, and the catalyst composition of claim 1.

9. The premix of claim 8, further comprising at least one additive selected from at least one cell stabilizer, at least one flame retardant, at least one chain extender, at least one epoxy resin, at least one acrylic resin, at least one filler, at least one pigment, or any combination thereof.

10. A method for preparing a polyurethane foam comprising contacting at least one polyisocyanate with at least one active hydrogen-containing compound in the presence of at least one blowing agent and the catalyst composition defined in claim 1.

11. The method of claim 10 wherein the catalyst composition is present in combination with a metal catalyst, a tertiary amine having or not an isocyanate reactive group, or a combination thereof.

## Patentansprüche

1. Katalysatorzusammensetzung, umfassend mindestens eine Verbindung, die aus der Gruppe bestehend aus N,N'-Dipropyl-N,N'-dimethylbis(aminoethyl)ether; N,N'-Diisopropyl-N,N'-dimethylbis(aminoethyl)ether; N,N'-Dibutyl-N,N'-dimethylbis(aminoethyl)ether; N,N'-Diisobutyl-N,N'-dimethylbis(aminoethyl)ether; N,N'-Di-sec-butyl-N,N'-dimethylbis(aminoethyl)ether; N,N'-Di-tert-butyl-N,N'-dimethylbis(aminoethyl)ether; N,N'-Dipentyl-N,N'-dimethylbis(aminoethyl)ether; N,N'-Di-tert-pentyl-N,N'-dimethylbis(aminoethyl)ether; N,N'-Dineopentyl-N,N'-dimethylbis(aminoethyl)ether; N,N'-Di-sec-pentyl-N,N'-dimethylbis(aminoethyl)ether; N,N'-Di(3-pentyl)-N,N'-dimethylbis(aminoethyl)ether; N,N'-Di-sec-isoopentyl-N,N'-dimethylbis(aminoethyl)ether; N,N'-Dihexyl-N,N'-dimethylbis(aminoethyl)ether; N,N'-Diisohexyl-N,N'-dimethylbis(aminoethyl)ether und N,N'-Dineohexyl-N,N'-dimethylbis(aminoethyl)ether ausgewählt ist.

2. Katalysatorzusammensetzung nach Anspruch 1, ferner umfassend einen Metallkatalysator, einen tertiären Aminkatalysator, der gegebenenfalls eine isocyanatreaktive Gruppe aufweist, oder eine Kombination davon.

3. Katalysatorzusammensetzung nach Anspruch 2, wobei das tertiäre Amin mindestens eine isocyanatreaktive Gruppe aufweist, die eine primäre Hydroxylgruppe, eine sekundäre Hydroxylgruppe, eine primäre Amingruppe, eine sekundäre Amingruppe, eine Harnstoffgruppe oder eine Amidgruppe umfasst.

4. Katalysatorzusammensetzung nach Anspruch 3, wobei das tertiäre Amin aus der Gruppe bestehend aus N,N-Bis(3-dimethylaminopropyl)-N-isopropanolamin; N,N-Dimethylaminoethyl-N'-methylethanolamin; N,N,N'-Trimethylaminopropylethanolamin; N,N-Dimethylethanolamin; N,N-Diethylethanolamin; N,N-Dimethyl-N',N'-2-hydroxy(propyl)-1,3-propylendiamin; Dimethylaminopropylamin; (N,N-Dimethylaminoethoxy)ethanol; Methylhydroxyethylpiperazin; Bis(N,N-dimethyl-3-aminopropyl)amin; N,N-Dimethylaminopropylharnstoff; Diethylaminopropylharnstoff; N,N'-Bis(3-dimethylaminopropyl)harnstoff; N,N'-Bis(3-diethylaminopropyl)harnstoff; Bis(dimethylamino)-2-propanol; 6-Dimethylamino-1-hexanol; N-(3-Aminopropyl)imidazol); N-(2-Hydroxypropyl)imidazol; N-(2-Hydroxyethyl)imidazol; 2-[N-(Dimethylaminoethoxyethyl)-N-methylamino]ethanol; N,N-Dimethylaminoethyl-N'-methyl-N'-ethanol; Dimethylaminoethoxyethanol; N,N,N'-Trimethyl-N'-3-aminopropyl-bis(aminoethyl)ether oder einer Kombination davon ausgewählt ist.

5. Zusammensetzung nach Anspruch 1, wobei die Katalysatorzusammensetzung mit einer Carbon- oder Sulfonsäure säureblockiert ist.

6. Katalysatorzusammensetzung nach Anspruch 5, wobei die Zusammensetzung mit einer Säure säureblockiert ist, die aus der Gruppe bestehend aus Ameisensäure, Essigsäure, Propionsäure, Butansäure, Pentansäure, Neopentansäure, Hexansäure, 2-Ethylhexylcarbonsäure, Neohexansäure, Octansäure, Neooctansäure, Heptansäure, Neoheptansäure, Nonansäure, Neononansäure, Decansäure, Neodecansäure, Undecansäure, Neoundecansäure, Dodecansäure, Neododecansäure, Myristinsäure, Pentadecansäure, Hexadecansäure, Heptadecansäure, Octadecansäure, Benzoesäure, Oxalsäure, Malonsäure, Bernsteinsäure, Glutarsäure, Adipinsäure, Pimelinsäure, Korksäure, Azelainsäure, Sebacinsäure, Glycolsäure, Milchsäure, Weinsäure, Citronensäure, Äpfelsäure und Salicylsäure ausgewählt ist.

7. Katalysatorzusammensetzung nach Anspruch 1, ferner umfassend katalytische Materialien, wobei die katalytischen Materialien aus der Gruppe bestehend aus Kaliumformiat, Kaliumacetat, Kaliumpropionat, Kaliumbutanoat, Kaliumpentanoat, Kaliumhexanoat, Kaliumheptanoat, Kaliumoctanoat, Kalium-2-ethylhexanoat, Kaliumdecanoat, Kaliumbutyrat, Kaliumisobutyrat, Kaliumnonanoat, Kaliumstearat, Natriumoctanoat, Lithiumstearat, Natriumcaprat, Lithiumoctanoat, 2-Hydroxypropyltrimethylammoniumoctanoat-Lösung oder einer beliebigen Kombination davon ausgewählt sind.

8. Vormischung, umfassend das Kontaktprodukt von mindestens einer aktiven Wasserstoff enthaltenden Verbindung, mindestens einem Treibmittel und der Katalysatorzusammensetzung nach Anspruch 1.

9. Vormischung nach Anspruch 8, ferner umfassend mindestens ein Additiv, das aus mindestens einem Zellstabilisator, mindestens einem Flammschutzmittel, mindestens einem Kettenverlängerer, mindestens einem Epoxidharz, mindestens einem Acrylharz, mindestens einem Füllstoff, mindestens einem Pigment oder einer beliebigen Kombination davon ausgewählt ist.

10. Verfahren zur Herstellung eines Polyurethanschaumstoffs, umfassend das Inkontaktbringen von mindestens einem Polyisocyanat mit mindestens einer aktiven Wasserstoff enthaltenden Verbindung in Gegenwart von mindestens einem Treibmittel und der in Anspruch 1 definierten Katalysatorzusammensetzung.

11. Verfahren nach Anspruch 10, wobei die Katalysatorzusammensetzung in Kombination mit einem Metallkatalysator, einem tertiären Amin, das gegebenenfalls eine isocyanatreaktive Gruppe aufweist, oder einer Kombination davon vorliegt.

## Revendications

1. Composition de catalyseur comprenant au moins un composé choisi dans le groupe constitué par N,N'-dipropyl-N,N'-diméthyl-bis(aminoéthyl)éther ; N,N'-diisopropyl-N,N'-diméthyl-bis(aminoéthyl)éther ; N,N'-dibutyl-N,N'-diméthyl-bis(aminoéthyl)éther ; N,N'-diisobutyl-N,N'-diméthyl-bis(aminoéthyl)éther ; N,N'-disecbutyl-N,N'-diméthyl-bis(aminoéthyl)éther ; N,N'-ditertbutyl-N,N'-diméthyl-bis(aminoéthyl)éther ; N,N'-dipentyl-N,N'-diméthyl-bis(aminoéthyl)éther ; N,N'-ditertpentyl-N,N'-diméthyl-bis(aminoéthyl)éther ; N,N'-dinéopentyl-N,N'-diméthyl-bis(aminoéthyl)éther ; N,N'-disecpentyl-N,N'-diméthyl-bis(aminoéthyl)éther ; N,N'-di(3-pentyl)-N,N'-diméthyl-bis(aminoéthyl)éther ; N,N'-disecisoopentyl-N,N'-diméthyl-bis(aminoéthyl)éther ; N,N'-dihexyl-N,N'-diméthyl-bis(aminoéthyl)éther ; N,N'-diisohexyl-N,N'-diméthyl-bis(aminoéthyl)éther ; et N,<N'-dinéohexyl-N,N'-diméthyl-bis(aminoéthyl)éther.

2. Composition de catalyseur selon la revendication 1, comprenant en outre un catalyseur métallique, un catalyseur d'amine tertiaire ayant ou non un groupe réactif envers un isocyanate, ou une combinaison correspondante.

3. Composition de catalyseur selon la revendication 2, dans laquelle l'amine tertiaire a au moins un groupe réactif envers un isocyanate comprenant un groupe hydroxyle primaire, un groupe hydroxyle secondaire, un groupe amine primaire, un groupe amine secondaire, un groupe urée ou un groupe amide.

4. Composition de catalyseur selon la revendication 3, l'amine tertiaire étant choisie dans le groupe constitué par N,N-bis(3-diméthylaminopropyl)-N-isopropanolamine ; N,N-diméthylaminoéthyl-N'-méthyléthanolamine ; N,N,N'-triméthylaminopropyléthanolamine ; N,N-diméthyléthanolamine ; N,N-diéthyléthanolamine ; N,N-diméthyl-N',N'-2-hydroxy(propyl)-1,3-propylènediamine ; diméthylaminopropylamine ; (N,N-diméthylaminoéthoxy)éthanol ; méthyl-hydroxy-éthyl-pipérazine ; bis(N,N-diméthyl-3-aminopropyl)amine ; N,N-diméthylaminopropylurée ; diéthylaminopropylurée ; N,N'-bis(3-diméthylaminopropyl)urée ; N,N'-bis(3-diéthylaminopropyl)urée ; bis(diméthylamino)-2-propanol ; 6-diméthylamino-1-hexanol ; N-(3-aminopropyl)imidazole) ; N-(2-hydroxypropyl)imidazole ; N-(2-hydroxyéthyl)imidazole ; 2-[N-(diméthylaminoéthoxyéthyl)-N-méthylamino]éthanol ; N,N-diméthylaminoéthyl-N'-méthyl-N'-éthanol ; diméthylaminoéthoxyéthanol ; N,N,N'-triméthyl-N'-3-aminopropyl-bis(aminoéthyl)éther ; ou une combinaison correspondante.

5. Composition de catalyseur selon la revendication 1, dans laquelle la composition de catalyseur est bloquée par un acide carboxylique ou sulfonique.

6. Composition de catalyseur selon la revendication 5, dans laquelle la composition est bloquée par un acide choisi dans le groupe constitué par acide formique, acide acétique, acide propionique, acide butanoïque, acide pentanoïque, acide néopentanoïque, acide hexanoïque, acide 2-éthylhexylcarboxylique, acide néohexanoïque, acide octanoïque, acide néooctanoïque, acide heptanoïque, acide néoheptanoïque, acide nonanoïque, acide néononanoïque, acide décanoïque, acide néodécanoïque, acide undécanoïque, acide néoundécanoïque, acide dodécanoïque, acide néododécanoïque, acide myristique, acide pentadécanoïque, acide hexadécanoïque, acide heptadécanoïque, acide octadécanoïque, acide benzoïque, acide oxalique, acide malonique, acide succinique, acide glutarique, acide adipique, acide pimélique, acide subérique, acide azélaïque, acide sébacique, acide glycolique, acide lactique, acide tartrique, acide citrique, acide malique et acide salicylique.

7. Composition de catalyseur selon la revendication 1, comprenant en outre des matériaux catalytiques, dans laquelle les matériaux catalytiques sont choisis dans le groupe constitué par formiate de potassium, acétate de potassium, propionate de potassium, butanoate de potassium, pentanoate de potassium, hexanoate de potassium, heptanoate de potassium, octoate de potassium, 2-éthylhexanoate de potassium, décanoate de potassium, butyrate de potassium, isobutyrate de potassium, nonante de potassium, stéarate de potassium, octoate de sodium, stéarate de lithium, caprioate de sodium, octoate de lithium, solution d'octoate de 2-hydroxypropyltriméthylammonium, ou toute combinaison correspondante.

8. Prémélange comprenant le produit de contact d'au moins un composé contenant un hydrogène actif, d'au moins un agent de gonflement et de la composition de catalyseur selon la revendication 1.

9. Prémélange selon la revendication 8, comprenant en outre au moins un additif choisi parmi au moins un stabilisant d'alvéoles, au moins un agent ignifugeant, au moins un extenseur de chaîne, au moins une résine époxy, au moins une résine acrylique, au moins une charge, au moins un pigment, ou toute combinaison correspondante.

10. Procédé de préparation d'une mousse de polyuréthane comprenant la mise en contact d'au moins un polyisocyanate avec au moins un composé contenant un hydrogène actif en présence d'au moins un agent gonflant et de la composition de catalyseur définie dans la revendication 1.

11. Procédé selon la revendication 10, dans lequel la composition de catalyseur est présente en combinaison avec un catalyseur métallique, une amine tertiaire ayant ou non un groupe réactif envers un isocyanate, ou une combinaison correspondante.
